(19) Europäisches Patentamt
European Patent Office
Office européen des brevets

(11) Veröffentlichungsnummer: **0 456 133 B1**

(12) **EUROPÄISCHE PATENTSCHRIFT**

(45) Veröffentlichungstag der Patentschrift: **15.03.95**

(51) Int. Cl.[6]: **C07C 281/12**, C07C 281/18, C07C 337/08, A61K 31/175

(21) Anmeldenummer: **91107225.4**

(22) Anmeldetag: **03.05.91**

# (54) Hydrazone.

(30) Priorität: **07.05.90 CH 1538/90**

(43) Veröffentlichungstag der Anmeldung:
**13.11.91 Patentblatt 91/46**

(45) Bekanntmachung des Hinweises auf die Patenterteilung:
**15.03.95 Patentblatt 95/11**

(84) Benannte Vertragsstaaten:
**AT BE CH DE DK ES FR GB GR IT LI LU NL SE**

(56) Entgegenhaltungen:
**EP-A- 0 163 888**
**EP-A- 0 377 304**
**US-A- 4 076 726**

**CHEMICAL ABSTRACTS, Band 105, Nr. 1, 7. Juli 1986, Seite 587, Zusammenfassung Nr. 6283c, Columbus, Ohio, US; P. GAN et al.: "Synthesis of indanones and tetralones as new potential schistosomicidals"**

**CHEMICAL ABSTRACTS, Band 84, Nr. 11, 15. März 1976, Seite 419, Zusammenfassung Nr. 73957m, Columbus, Ohio, US; V.S. MISRA et al.: "Synthesis and antibacterial activity of thiosemicarbazones and hydrazones derived from indan-1-one"**

(73) Patentinhaber: **CIBA-GEIGY AG**
**Klybeckstrasse 141**
**CH-4002 Basel (CH)**

(72) Erfinder: **Stanek, Jaroslav, Dr.**
**Hangstrasse 9**
**CH-4144 Arlesheim (CH)**
Erfinder: **Frei, Jörg, Dr.**
**Buechring 36**
**CH-4434 Hölstein (CH)**
Erfinder: **Caravatti, Giorgio, Dr.**
**Birnbaumweg 13**
**CH-4103 Bottmingen (CH)**

(74) Vertreter: **Zumstein, Fritz, Dr. et al**
**Patentanwälte,**
**Dr. F. Zumstein,**
**Dipl.-Ing. F. Klingseisen,**
**Bräuhausstrasse 4**
**D-80331 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 456 133 B1

## Beschreibung

Die Erfindung betrifft Verbindungen der Formel I

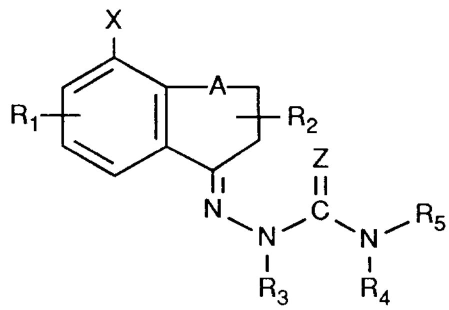

(I)

worin A eine direkte Bindung oder $-(CH_2)_n-$, worin n für 1, 2 oder 3 steht, bedeutet; X für einen Rest -C(=Y)-$NR_6R_7$ steht; Y für $NR_8$, O oder S steht; Z für $NR_9$, O oder S steht; $R_1$ und $R_2$ unabhängig voneinander Wasserstoff oder einen oder mehrere Substituenten ausgewählt aus Niederalkyl, Trifluormethyl, $C_3$-$C_8$-Cycloalkyl, welches unsubstituiert oder durch Niederalkyl substituiert ist, Arylniederalkyl, Hydroxy, Niederalkoxy, Arylniederalkoxy, Aryloxy, Niederalkanoyloxy, Halogen, Amino, N-Niederalkylamino, N,N-Diniederalkylamino, Niederalkanoylamino, Nitro, Niederalkanoyl, Arylcarbonyl, Carboxy, Niederalkoxycarbonyl, Carbamoyl, N-Niederalkylcarbamoyl, N,N-Diniederalkylcarbamoyl, N-Arylcarbamoyl, Cyano, Mercapto, Niederalkylthio, Niederalkylsulfonyl, Sulfamoyl, N-Niederalkylsulfamoyl und N-N-Diniederalkylsulfamoyl bedeuten; die Reste $R_3$, $R_4$, $R_6$, $R_8$ und $R_9$ unabhängig voneinander Wasserstoff oder Niederalkyl bedeuten; und $R_5$ und $R_7$ unabhängig voneinander für Wasserstoff, Niederalkyl, Hydroxy, Niederalkoxy, Niederalkanoyloxy, unsubstituiertes Amino, Diniederalkylamino, $C_4$-$C_7$-Alkylenamino oder Oxa-, Thia- oder Aza-$C_4$-$C_7$-alkylenamino stehen; Tautomere davon, oder Salze davon; wobei Aryl für Phenyl oder Naphthyl steht, welches jeweils unsubstituiert oder durch einen oder mehrere Substituenten ausgewählt aus Niederalkyl, Niederalkoxy, Hydroxy, Niederalkanoyloxy, Nitro, Amino, Halogen, Trifluormethyl, Carboxy, Niederalkoxycarbonyl, Carbamoyl, N-Niederalkylcarbamoyl, N,N-Diniederalkylcarbamoyl, Cyano, Niederalkanoyl, Arylcarbonyl, Niederalkylsulfonyl, Sulfamoyl, N-Niederalkylsulfamoyl und N,N-Diniederalkylsulfamoyl substituiert ist; und wobei "Nieder" oder "nieder" einen Rest bis und mit maximal 7 Kohlenstoffatomen bezeichnet.

Tautomere können z.B. dann auftreten, wenn Z für $NR_9$ steht und $R_3$ und/oder $R_4$ und/oder $R_5$ Wasserstoff bedeuten:
Der entsprechende Guanylrest, in Formel I als $-N(R_3)-C(=Z)-NR_4R_5$ dargestellt, kann dann z.B. auch in den tautomeren Formen $-N=C(-ZH)-NR_4R_5$, $-N(R_3)-C(-ZH)=NR_5$ oder $-N(R_3)-C(-ZH)=NR_4$ vorliegen.
Ein weiteres Beispiel: Steht Y für $NR_8$ und ist $R_6$ und/oder $R_7$ Wasserstoff, so kann die entsprechende Amidinstruktur, in Formel I als X = $-C(=Y)-NR_6R_7$ definiert, ebenso in den tautomeren Formen $-C(-YH)=NR_7$ oder $-C(-YH)=NR_6$ auftreten. Dem Fachmann ist das Vorliegen solcher und ähnlicher Tautomere geläufig. Alle diese Tautomere werden von der allgemeinen Formel I mit umfasst.

Im Falle, dass A eine Gruppe $-(CH_2)_n-$ bedeutet und $R_2$ von Wasserstoff verschieden ist, können der oder die dem Rest $R_2$ entsprechenden Substituenten auch an den Kohlenstoffatomen der Gruppe $-(CH_2)_n-$ angeknüpft sein.

$R_2$ steht z.B. für Wasserstoff oder 1-4 von Wasserstoff verschiedene Substituenten, insbesondere für Wasserstoff oder 1-2 von Wasserstoff verschiedene Substituenten und vor allen Dingen für Wasserstoff oder einen von Wasserstoff verschiedenen Substituenten.

$R_1$ steht z.B. für Wasserstoff oder 1-3 von Wasserstoff verschiedene Substituenten und insbesondere für Wasserstoff oder 1-2 von Wasserstoff verschiedene Substituenten.

Die vor- und nachstehend verwendeten Allgemeinbegriffe haben im Rahmen der vorliegenden Anmeldung vorzugsweise die folgenden Bedeutungen:
Das Präfix "Nieder" bezeichnet einen Rest bis und mit 7 und insbesondere bis und mit 4 Kohlenstoffatomen.

Niederalkyl ist z.B. n-Propyl, Isopropyl, n-Butyl, Isobutyl, sek-Butyl, tert-Butyl, n-Pentyl, Neopentyl, n-Hexyl oder n-Heptyl, vorzugsweise Ethyl und vor allem Methyl.

Aryl ist Phenyl oder Naphthyl, wie 1- oder 2-Naphthyl. Die Phenyl- und Naphthylreste können unsubstituiert oder substituiert sein, insbesondere wie nachfolgend für Phenyl angegeben. Aryl ist bevorzugt Phenyl, das unsubstituiert oder durch einen oder mehrere, insbesondere einen oder zwei, Substituenten aus

der Gruppe bestehend aus Niederalkyl, Niederalkoxy, Hydroxy, Niederalkanoyloxy, Nitro, Amino, Halogen, Trifluormethyl, Carboxy, Niederalkoxycarbonyl, Carbamoyl, N-Niederalkylcarbamoyl, N,N-Diniederalkylcarbamoyl, Cyano, Niederalkanoyl, Arylcarbonyl, Niederalkylsulfonyl, Sulfamoyl, N-Niederalkylsulfamoyl und N,N-Diniederalkylsulfamoyl substituiert ist. In erster Linie bedeutet Aryl Phenyl, das unsubstituiert oder durch Niederalkyl, Niederalkoxy, Hydroxy, Halogen oder Trifluormethyl substituiert ist, und vor allen Dingen Phenyl.

Arylcarbonyl ist z.B. Benzoyl, welches unsubstituiert oder durch Niederalkyl, Niederalkoxy, Hydroxy, Halogen oder Trifluormethyl substituiert ist, und insbesondere Benzoyl.

Arylniederalkyl ist z.B. Phenylniederalkyl und insbesondere Benzyl.

Halogen steht insbesondere für Chlor und Brom, kann aber auch Fluor oder Iod bedeuten.

Niederalkanoyl ist z.B. Formyl, Acetyl, Propionyl oder Pivaloyl.

Cycloalkyl ist $C_3$-$C_8$- und insbesondere $C_5$-$C_6$-Cycloalkyl, was bedeuten soll, dass es 3 bis 8 bzw. 5 bis 6 Ringkohlenstoffe enthält. Es kann aber auch substituiert sein durch Niederalkyl.

Niederalkoxy, Niederalkanoyloxy, Niederalkylamino, Diniederalkylamino, $C_4$-$C_7$-Alkylenamino, insbesondere $C_4$-$C_5$-Alkylenamino, z.B. Piperidino, oder Oxa-, Thia- oder Azaniederalkylenamino, z.B. Morpholino, Thiomorpholino, Piperazino oder 4-Niederalkylpiperazino, können für $R_5$ und/oder $R_7$ stehen.

Salze von erfindungsgemässen Verbindungen sind in erster Linie pharmazeutisch verwendbare, nichttoxische Salze. Beispielsweise können Verbindungen der Formel I mit basischen Gruppen Säureadditionssalze, z.B. mit anorganischen Säuren, wie Salzsäure, Schwefelsäure oder Phosphorsäure, oder mit geeigneten organischen Carbon- oder Sulfonsäuren, z.B. Essigsäure, Fumarsäure oder Methansulfonsäure, oder z.B. mit Aminosäuren, wie Arginin oder Lysin, bilden. Bei Anwesenheit von mehreren basischen Gruppen können Mono- oder Polysalze gebildet werden. Verbindungen der Formel I mit einer sauren Gruppe, z.B. Carboxy, und einer basischen Gruppe, z.B. Amino, können z.B. in Form von inneren Salzen, d.h. in zwitterionischer Form vorliegen, oder es kann ein Teil des Moleküls als inneres Salz, und ein anderer Teil als normales Salz vorliegen.

Zur Isolierung oder Reinigung können auch pharmazeutisch ungeeignete Salze, z.B. Pikrate oder Perchlorate, Verwendung finden. Zur therapeutischen Anwendung gelangen nur die pharmazeutisch verwendbaren, nicht-toxischen Salze, die deshalb bevorzugt sind.

Je nach den strukturellen Gegebenheiten können die Verbindungen der vorliegenden Erfindung in Form von Isomerengemischen oder von reinen Isomeren vorliegen. Steht z.B. $R_2$ für einen von Wasserstoff verschiedenen Substituenten, so können die entsprechenden Verbindungen der Formel I als Racemate oder reine Enantiomere vorliegen.

Die erfindungsgemässen Verbindungen weisen wertvolle, insbesondere pharmakologisch verwendbare, Eigenschaften auf. Insbesondere haben sie eine starke, spezifische Hemmwirkung auf das Enzym S-Adenosylmethionindecarboxylase (SAMDC). SAMDC spielt als ein Schlüsselenzym eine wichtige Rolle bei der Polyaminsynthese, die in praktisch allen Zellen von Säugetieren, einschliesslich Menschen, abläuft. Durch SAMDC wird die Polyaminkonzentration in der Zelle reguliert. Eine Hemmung des Enzyms SAMDC hat eine Verringerung der Polyaminkonzentration zur Folge. Da eine Verringerung der Polyaminkonzentration eine Hemmung des Zellwachstums bewirkt, ist es möglich, durch Verabreichung von SAMDChemmenden Substanzen das Wachstum sowohl von eukaryotischen als auch prokaryotischen Zellen zu hemmen und sogar Zellen abzutöten oder das Einsetzen der Zelldifferenzierung zu hemmen.

Die Hemmung des Enzyms SAMDC kann z.B. mit der Methode von H.G. Williams-Ashmann und A. Schenone, Biochem. Biophys. Res. Communs. 46, 288 (1972) nachgewiesen werden. Die Verbindungen der Erfindung weisen $IC_{50}$-Werte von minimal etwa 0.005 $\mu$M auf.

Ein weiterer Vorteil der erfindungsgemässen Verbindungen besteht darin, dass sie nur in geringem Masse im Vergleich zu ihrer starken Hemmwirkung auf SAMDC die Diaminoxidase inhibieren und gut verträglich sind. Die Hemmungen der Diaminoxidase ist nach J. Jaenne und D.R. Morris, Biochem. J. 218, 974 (1984) ungünstig, da sie zur Akkumulation von Putrescin und einer indirekten SAMDC-Aktivierung führen kann.

Daher sind die Verbindungen der Formel I z.B. nützlich zur Behandlung benigner und maligner Tumoren. Sie können Tumorregressionen bewirken und ferner die Verbreitung von Tumorzellen sowie das Wachstum von Mikrometastasen verhindern. Des weiteren können sie z.B. zur Behandlung von Protozoainfektionen, wie etwa Trypanosomiasis, Malaria oder durch Pneumocystis carinii verursachte Lungenentzündung, dienen.

Als selektive SAMDC-Hemmer können die Verbindungen der Formel I allein oder auch in Kombination mit anderen pharmakologisch wirksamen Substanzen angewendet werden. Zu denken ist z.B. an eine Kombination mit (a) Inhibitoren anderer Enzyme der Polyaminbiosynthese, z.B. Ornithindecarboxylasehemmern, (b) Inhibitoren der Proteinkinase C, (c) Inhibitoren der Tyrosinproteinkinase, (d) Cytokinen, (e)

negativen Wachstumsregulatoren, (f) Aromatasehemmern, (g) Antiöstrogenen oder (h) klassischen zytostatischen Wirkstoffen.

Bevorzugt betrifft die Erfindung die Verbindungen der Formel I, worin A eine direkte Bindung oder $-(CH_2)_n-$, worin n für 1 oder 2 steht, bedeutet; X für einen Rest $-C(=Y)-NR_6R_7$ steht; Y für $NR_8$, O oder S steht; Z für $NR_9$, O oder S steht; $R_1$ und $R_2$ unabhängig voneinander Wasserstoff oder einen oder zwei Substituenten aus der Gruppe bestehend aus Niederalkyl, Trifluormethyl, Cycloalkyl, Arylniederalkyl, Hydroxy, Niederalkoxy, Arylniederalkoxy, Aryloxy, Niederalkanoyloxy, Halogen, Amino, N-Niederalkylamino, N,N-Diniederalkylamino, Niederalkanoylamino, Nitro, Niederalkanoyl, Arylcarbonyl, Carboxy, Niederalkoxycarbonyl, Carbamoyl, N-Niederalkylcarbamoyl, N,N-Diniederalkylcarbamoyl, N-Arylcarbamoyl, Cyano, Mercapto, Niederalkylthio, Niederalkylsulfonyl, Sulfamoyl, N-Niederalkylsulfamoyl und N,N-Diniederalkylsulfamoyl bedeuten, wobei Aryl für Phenyl steht, das unsubstituiert oder durch Niederalkyl, Niederalkoxy, Hydroxy, Halogen oder Trifluormethyl substituiert ist; die Reste $R_3$, $R_4$, $R_6$, $R_8$ und $R_9$ unabhängig voneinander Wasserstoff oder Niederalkyl bedeuten; und $R_5$ und $R_7$ unabhängig voneinander für Wasserstoff, Niederalkyl, Hydroxy, Niederalkoxy, Niederalkanoyloxy, Amino, Niederalkylamino, Diniederalkylamino, Niederalkylenamino oder Oxa-, Thia- oder Azaniederalkylenamino stehen; Tautomere davon, und Salze davon.

Besonders bevorzugt sind die Verbindungen der Formel I, worin A eine direkte Bindung oder $-(CH_2)_n-$, worin n für 1 oder 2 steht, bedeutet; X für einen Rest $-C(=Y)-NR_6R_7$ steht; Y für NH, O oder S steht; Z für NH, O oder S steht; $R_1$ und $R_2$ unabhängig voneinander Wasserstoff oder einen oder zwei Substituenten aus der Gruppe bestehend aus Niederalkyl, Trifluormethyl, Phenylniederalkyl, Hydroxy, Niederalkoxy und Halogen bedeuten; die Reste $R_3$, $R_4$ und $R_6$ Wasserstoff bedeuten; und $R_5$ und $R_7$ unabhängig voneinander für Wasserstoff, Niederalkyl, Hydroxy oder Amino stehen; Tautomere davon, oder Salze davon.

In erster Linie bevorzugt sind die Verbindungen der Formel I, worin A eine direkte Bindung oder $-CH_2-$ bedeutet; X für einen Rest $-C(=Y)-NR_6R_7$ steht; Y für NH oder S steht; Z für NH steht; $R_1$ Wasserstoff oder einen oder zwei Substituenten aus der Gruppe bestehend aus Niederalkyl, Hydroxy, Niederalkoxy und Halogen bedeutet; $R_2$ für Wasserstoff oder Niederalkyl steht; die Reste $R_3$, $R_4$ und $R_6$ Wasserstoff bedeuten; und $R_5$ und $R_7$ unabhängig voneinander für Wasserstoff, Niederalkyl oder Hydroxy stehen; Tautomere davon, oder pharmazeutisch anwendbare Salze davon.

Vor allem bevorzugt sind die Verbindungen der Formel I, worin A eine direkte Bindung bedeutet, X für einen Rest $-C(=NH)-NH_2$ steht; Z für NH steht; $R_1$ Wasserstoff oder einen oder zwei Substituenten aus der Gruppe bestehend aus Niederalkyl, Hydroxy und Niederalkoxy bedeutet; die Reste $R_2$, $R_3$ und $R_4$ Wasserstoff bedeuten; und $R_5$ für Wasserstoff oder Hydroxy steht; Tautomere davon, oder pharmazeutisch anwendbare Salze davon.

Als Untergruppen aus einer Gruppe von Verbindungen der Formel I sind jeweils hervorzuheben: (a) Verbindungen der Formel I, worin A eine direkte Bindung bedeutet; (b) Verbindungen der Formel I, worin X für einen Rest $-C(=NH)-NH_2$ steht; (c) Verbindungen der Formel I, worin Z für NH steht, $R_4$ Wasserstoff bedeutet, und $R_5$ für Wasserstoff oder Hydroxy steht; und (d) Verbindungen der Formel I, worin $R_1$ und $R_2$ Wasserstoff bedeuten.

Die Erfindung betrifft vor allem die in den Beispielen beschriebenen spezifischen Verbindungen oder Salze davon.

Die Verbindungen der Formel I können in an sich bekannter Weise hergestellt werden, indem man z.B.

(a) eine Verbindung der Formel II

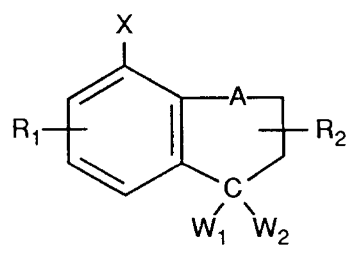

(II)

worin die Gruppe $CW_1W_2$ für Carbonyl, funktionell abgewandeltes Carbonyl oder geschütztes Carbonyl steht und A, X, $R_1$ und $R_2$ die unter Formel I angegebene Bedeutung haben, mit einem Amin der Formel III

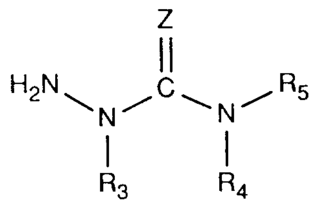

(III)

worin Z, $R_3$, $R_4$ und $R_5$ wie unter Formel I definiert sind, kondensiert, oder
(b) in einer Verbindung der Formel IV

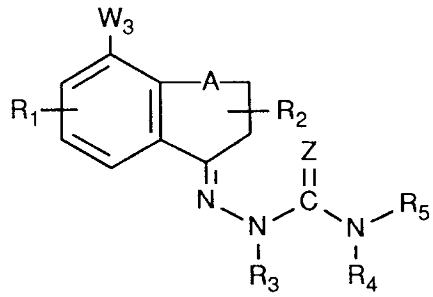

(IV)

worin $W_3$ einen Rest, der in eine Gruppe X der Formel I überführt werden kann, bedeutet und A, Z, $R_1$, $R_2$, $R_3$, $R_4$ und $R_5$ wie unter Formel I definiert sind, den Rest $W_3$ in die Gruppe X überführt; und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt.

In der folgenden näheren Beschreibung der Verfahren a)-b) haben die Symbole A, X, Y, Z und $R_1$-$R_9$ jeweils die unter Formel I angegebene Bedeutung, sofern nichts anderes angegeben ist.

Verfahren (a):

Als funktionell abgewandeltes bzw. geschütztes Carbonyl $CW_1W_2$ sind beispielhaft zu nennen: Diniederalkoxymethyl, $C_1$-$C_2$-Alkylendioxymethyl, Dihalogenmethyl, Diniederalkylthiomethyl oder $C_1$-$C_2$-Alkylendithiomethyl.

Bevorzugt liegt die Gruppe $CW_1W_2$ in den Verbindungen der Formel II als freies Carbonyl vor.

Die Kondensationsreaktion gemäss Verfahren (a) erfolgt unter den an sich bekannten Bedingungen bei der Bildung von Hydrazonen. Sie ist bevorzugt durch Säure katalysiert. In Verbindungen der Formel II sind solche geschützte Carbonylgruppen $CW_1W_2$ geeignet, die unter den Bedingungen der Kondensation in freies Carbonyl übergehen.

Zur Herstellung von Verbindungen der Formel I, worin $R_5$ Amino bedeutet, empfiehlt es sich, die Verbindung der Formel III im Ueberschuss einzusetzen.

Die Zwischenprodukte der Formel II, worin Y im Rest X NH bedeutet, werden z.B. dadurch erhalten, dass man eine Verbindung der Formel V

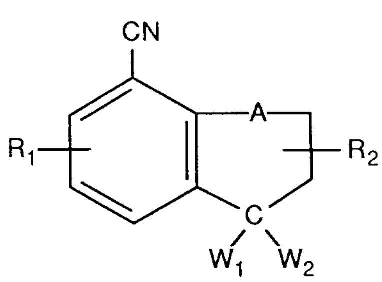

(V)

zunächst durch Behandlung mit Schwefelwasserstoff in das entsprechende Thiocarboxamid [-C(=S)-$NH_2$] überführt. Letzteres kann auch auf anderem Wege ausgehend vom analogen Carboxamid [-C(=O)-$NH_2$]

z.B. durch Umsetzung mit dem Lawesson-Reagens [2,4-Bis-(4-methoxyphenyl)-2,4-dithioxo-1,3,2,4-dithiadiphosphetan] erhalten werden. Die Thiocarboxamide werden z.B. mit Niederalkyliodid oder Triniederalkyloxoniumtetrafluoroborat S-alkyliert und damit in Imino-niederalkylthiolester-Hydroiodide [-C(=NH)-S-Alkyl•HI] bzw. -tetrafluoroborate überführt, welche sich leicht durch Umsetzung mit Ammoniak bzw. Aminen der Formel $NHR_6R_7$ in die gewünschten Carboximidamide der Formel II überführen lassen [vgl. S. Patai (Ed.), The Chemistry of amidines and imidates, Wiley, London etc. 1975, S. 303-304].

Die Herstellung der Carboxamide der Formel II aus den Cyanoverbindungen der Formel V verläuft analog der unten beim Verfahren (b) beschriebenen Herstellung von Carboxamiden der Formel I aus Cyanoverbindungen der Formel IV und ist dort detailliert beschrieben.

Eine weitere Möglichkeit zur Herstellung der Verbindungen der Formel II besteht darin, dass man eine Verbindung der Formel V, worin die Gruppe $CW_1W_2$ wie unter Formel II definiert ist, z.B. mit Ethanol und Salzsäure in z.B. Chloroform oder Diethylether behandelt, wobei das entsprechende Iminoethylester-Hydrochlorid gebildet wird, welches z.B. durch Umsetzung mit Ammoniak oder einem primären oder sekundären Amin der Formel $NHR_6R_7$ und z.B. Methanol in das gewünschte Carboximidamid der Formel II überführt werden kann. Diese Methode kann jedoch in einigen Fällen an sterischer Hinderung durch die Gruppen A bzw. $R_1$ scheitern.

Die Ausgangsverbindungen der Formel V sind an sich bekannt oder werden analog zu den bekannten Verbindungen hergestellt.

Die Verbindungen der Formel V lassen sich z.B. durch intramolekulare Friedel-Crafts-Acylierung von ω-Phenylniederalkansäuren der Formel VI,

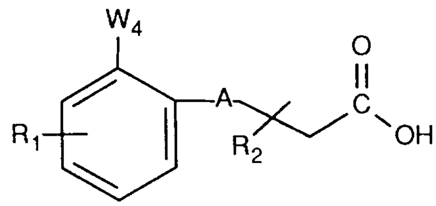

(VI)

worin $W_4$ Cyano oder eine Cyanovorstufe bedeutet, oder Säurederivaten davon, z.B. Säurechloriden oder Säureanhydriden, herstellen. Als Katalysatoren können bei freien Säuren z.B. Polyphosphorsäure und bei Säurechloriden oder -anhydriden z.B. $AlCl_3$ verwendet werden.

Bevorzugt werden bei dieser Reaktion Verbindungen der Formel VI eingesetzt, worin $W_4$ nicht Cyano, sondern eine Cyanovorstufe, z.B. Halogen, insbesondere Brom, oder geschütztes Amino, z.B. Acetylamino, bedeutet. Nach dem Cyclisierungsschnitt können dann die Cyanovorstufen in an sich bekannter Weise in Cyano überführt werden, z.B. Brom durch Umsetzung mit Kupfer(I)cyanid oder Acerylamino durch Abspaltung der Acetylschutzgruppe, Diazotierung und Umsetzung mit Kupfer(I)cyanid.

Verbindungen der Formel V, worin die Gruppe $CW_1W_2$ Carbonyl bedeutet, lassen sich ferner z.B. durch Oxidation, z.B. mit Chromtrioxid ($CrO_3$), aus den entsprechenden Nichtcarbonylverbindungen der Formel VII

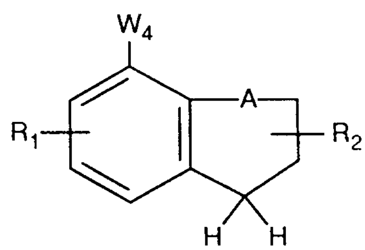

(VII)

worin $W_4$ Cyano oder eine Cyanovorstufe wie oben definiert bedeutet, herstellen. Wird eine Cyanovorstufe eingesetzt, so ist diese wiederum nach erfolgter Oxidation in Cyano zu überführen, z.B. wie oben angegeben.

Eine weitere Möglichkeit zur Herstellung der Verbindungen der Formel V, worin die Gruppe $CW_1W_2$ Carbonyl bedeutet, besteht darin, dass man von Verbindungen der Formel II, worin X Wasserstoff bedeutet, ausgeht und die Cyanogruppe einführt, beispielsweise durch eine Reaktionssequenz analog US-Patent 3,956,363, Beispiel 10, die aus Nitrierung, Reduktion der Nitrogruppe zu Amino, Diazotierung und Umsetzung mit Kupfer(I)cyanid (Sandmeyer-Reaktion) besteht.

Die Herstellung von Aminoguanidinen, -harnstoffen und -thioharnstoffen der Formel III ist an sich bekannt. Amino(thio)harnstoffe [≙ Semi(thio)carbazide] werden z.B. in analoger Weise hergestellt wie entsprechende einfache (Thio-)Harnstoffe. Dabei werden z.B. anstelle von Aminen die entsprechenden Hydrazine der Formel $H_2N\text{-}NHR_3$ eingesetzt und z.B. mit einem Isocyanat der Formel $R_4N=C=O$ oder $R_5N=C=O$, einem Isothiocyanat der Formel $R_4N=C=S$ oder $R_5N=C=S$, einem Carbamoylchlorid der Formel $R_4R_5N\text{-}COCl$ oder einem Thiocarbamoylchlorid der Formel $R_4R_5N\text{-}CSCl$ umgesetzt. Weiterhin ist z.B. auch die Reaktion eines Hydrazins der Formel $H_2N\text{-}NHR_3$ mit einem Acylisothiocyanat, z.B. Acetylisothiocyanat, und nachfolgender saurer Hydrolyse möglich.

Aminoguanidine der Formel III, worin Z für $NR_9$ steht und $R_3$, $R_4$, $R_5$ und $R_9$ wie unter Formel I definiert sind, sind an sich bekannt und lassen sich z.B. aus entsprechenden Aminothioharnstoffen der Formel III herstellen, indem man letztere durch Alkylierung, etwa mit einem Alkyltosylat oder -halogenid, in die entsprechenden S-Alkylisothiuroniumsalze überführt und diese mit einem Amin der Formel $NHR_4R_5$ umsetzt.

<u>Verfahren (b):</u>

In den Zwischenprodukten der Formel IV bedeutet $W_3$ z.B. freies oder funktionell abgewandeltes Carboxy, insbesondere Halogencarbonyl, Cyan, Imino-niederalkoxycarbonyl oder Imino-niederalkylthiolcarbonyl.

Die Gruppe $W_3$ in einer Verbindung der Formel IV kann bei der Herstellung von Amidinen der Formel I (Y≙ $NR_8$) z.B. bedeuten: ein Säureadditionssalz eines Iminoniederalkylesters (≙ Iminoniederalkylether) oder Imino-niederalkylthiolesters, z.B. $-C(=NH)\text{-}OC_2H_5 \cdot HCl$ bzw. $-C(=NH)\text{-}SC_2H_5 \cdot HI$, oder Cyano.

Durch die Umsetzung eines Imino-niederalkylesters der Formel IV (als Salz) mit Ammoniak oder primären bzw. sekundären Aminen erhält man die unsubstituierten bzw. mono-oder disubstituierten Amidine der Formel I. Cyanoverbindungen der Formel IV können z.B. durch Umsetzung mit einem Alkalimetallamid, z.B. $KNH_2$, oder durch Reaktion mit einem primären oder sekundären (Di-)niederalkylammoniumhalogenid, z.B. $^{\oplus}NH_3CH_3Cl^{\ominus}$, in ein gegebenfalls mono- oder disubstituiertes Amidin der Formel I überführt werden.

Die Gruppe $W_3$ in einer Verbindung der Formel IV kann bei der Herstellung von Carbamoylverbindungen der Formel I (Y≙O) z.B. bedeuten: Carboxy, Halocarbonyl (z.B. -COCl), Niederalkoxycarbonyl oder Cyano. Die Bildung von gegebenenfalls mono- oder disubstituierten Carbamoylverbindungen der Formel I aus entsprechenden Zwischenprodukten der Formel IV, worin $W_3$ Carboxy, Halocarbonyl oder Niederalkoxycarbonyl bedeutet, durch Umsetzung mit Ammoniak bzw. primären oder sekundären Aminen ist an sich bekannt. Zwischenprodukte der Formel IV, worin $W_3$ Cyano bedeutet, können z.B. durch partielle Hydrolyse, im Sinne einer Graf-Ritter-Reaktion, oder über Carbonsäureesterimid-Salze in gegebenenfalls mono- oder disubstituierte Carbamoylverbindungen der Formel I überführt werden. Die Bedingungen bei der Hydrolyse der Cyano-Zwischenprodukte können so gewählt werden, dass die Reaktion auf der Stufe des Amids abgebrochen wird. Zu diesem Zweck kommt insbesondere die Hydrolyse mit Säuren, z.B. mit 80 %iger Schwefelsäure (unter Erwärmen), Polyphosphorsäure (bei 110-150 °C), Bromwasserstoff/Eisessig (bei Raumtemperatur, in Gegenwart von Ameisensäure oder ohne weiteres Lösungsmittel) oder HCl-Gas in etherischer Lösung gefolgt von der Zugabe von Wasser oder wässriger Salzsäure, oder die Umsetzung mit Borhalogeniden in Frage.

Mit Hilfe der Graf-Ritter-Reaktion gelingt auch die Herstellung N-substituierter Amide aus Nitrilen der Formel IV. Hierzu setzt man die Nitrile in Gegenwart einer starken Säure, vornehmlich 85-90 %iger Schwefelsäure, oder auch Polyphosphorsäure, Ameisensäure, Bortrifluorid oder anderen Lewis-Säuren, nicht jedoch Aluminiumchlorid, mit Verbindungen um, die in dem sauren Medium Carbeniumionen bilden können, also z.B. mit Olefinen, wie Propylen, oder Alkoholen, wie Ethanol.

Die Carbonsäureesterimide erhält man z.B. durch säurekatalysierte Anlagerung von Alkoholen an die Nitrile der Formel IV. Aus den Esterimiden erhält man die Amide im Sinne einer Pinner-Spaltung durch thermischen Zerfall der Esterimid-Salze bei Temperaturen oberhalb von etwa 80 °C.

Verbindungen der Formel IV, worin $W_3$ Cyano bedeutet, können z.B. dadurch hergestellt werden, dass man eine Verbindung der Formel V mit einer Verbindung der Formel III gemäss dem Verfahren a) umsetzt. Aus Verbindungen der Formel IV, worin $W_3$ Cyano bedeutet, lassen sich die anderen Verbindungen der Formel IV, worin $W_3$ freies oder anderweitig funktionell abgewandeltes Carboxy bedeutet, in an sich bekannter Weise oder wie oben beschrieben herstellen.

Verbindungen der Formel I können in andere Verbindungen der Formel I überführt werden.

So können z.B. Verbindungen der Formel I, worin X einen Rest $-C(=S)\text{-}NH_2$ bedeutet, durch S-Alkylierung, z.B. mit Triniederalkyloxonium-tetrafluoroborat, und anschliessende Umsetzung mit Ammoniak

bzw. einem Amin der Formel $NHR_6R_7$ oder insbesondere einem entsprechenden Ammoniumsalz davon in Verbindungen der Formel I, worin X für einen Rest -C(=NH)-$NR_6R_7$ steht, umgewandelt werden.

Verbindungen der Formel I, worin X für einen N-Hydroxyamidinorest -C(=$NR_8$)-NHOH steht, können z.B. durch Umsetzung mit Eisenpentacarbonyl [$Fe(CO)_5$] in andere Verbindungen der Formel I, worin X für einen analogen Amidinorest -C(=$NR_8$)-$NH_2$ steht, umgewandelt werden (siehe z.B. J. Chem. Soc. Chem. Commun. 1975, 761).

Verfahrensgemäss erhältliche freie Verbindungen der Formel I mit salzbildenden Eigenschaften können in an sich bekannter Weise in ihre Salze überführt werden, Verbindungen mit basischen Eigenschaften z.B. durch Behandeln mit Säuren oder geeigneten Derivaten davon, Verbindungen mit sauren Eigenschaften z.B. durch Behandeln mit Basen oder geeigneten Derivaten davon.

Infolge der engen Beziehung zwischen den Verbindungen der Formel I in freier Form und in Form von Salzen sind vor- und nachstehend unter den freien Verbindungen bzw. ihren Salzen sinn- und zweckmässig gegebenenfalls auch die entsprechenden Salze bzw. freien Verbindungen zu verstehen.

Die Verbindungen, einschliesslich ihrer Salze, können auch in Form von Hydraten erhalten werden, oder ihre Kristalle können z.B. das zur Kristallisation verwendete Lösungsmittel einschliessen.

Erfindungsgemäss erhältliche Gemische von Isomeren können in an sich bekannter Weise in die einzelnen Isomeren aufgetrennt werden, Racemate z.B. durch Bilden von Salzen mit optisch reinen salzbildenden Reagentien und Auftrennen des so erhältlichen Diastereomerengemisches, z.B. mittels fraktionierter Kristallisation.

Die oben angeführten Reaktionen können unter an sich bekannten Reäktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neuträlisierenden Agentien, je nach Art der Reaktion und/oder der Reaktionsteilnehmer bei erniedrigter, normaler oder erhöhter Temperatur, z.B. im Temperaturbereich von etwa -70°C bis etwa 190°C, vorzugsweise von etwa -20°C bis etwa 150°C, z.B. beim Siedepunkt des verwendeten Lösungsmittels, unter atmosphärischem Druck oder in einem geschlossenen Gefäss, gegebenenfalls unter Druck, und/oder in einer inerten Atmosphäre, z.B. unter einer Stickstoffatmosphäre.

Im Verfahren der vorliegenden Erfindung werden vorzugsweise solche Ausgangsstoffe eingesetzt, die zu den eingangs als besonders wertvoll beschriebenen Verbindungen führen.

Verwendbar sind auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsstoff unter den Reaktionsbedingungen gebildet oder in Form eines Derivates, z.B. eines Salzes davon, verwendet wird.

Die vorliegende Erfindung betrifft ebenfalls pharmazeutische Präparate, die als Wirkstoff eine der pharmakologisch wirksamen Verbindungen der Formel I enthalten. Besonders bevorzugt sind Präparate zur enteralen, insbesondere oralen, sowie zur parenteralen Verabreichung. Die Präparate enthalten den Wirkstoff allein oder vorzugsweise zusammen mit einem pharmazeutisch anwendbaren Trägermaterial. Die Dosierung des Wirkstoffs hängt von der zu behandelnden Krankheit, sowie von der Spezies, deren Alter, Gewicht und individuellem Zustand, sowie von der Applikationsweise ab.

Die pharmazeutischen Präparate enthalten von etwa 0,1 % bis etwa 95 % des Wirkstoffs, wobei einzeldosierte Applikationsformen vorzugsweise von etwa 1 % bis etwa 90 % und nicht-einzeldosierte Applikationsformen vorzugsweise etwa 0,1 % bis etwa 20 % Wirkstoff aufweisen. Dosiseinheitsformen, wie Dragées, Tabletten oder Kapseln, enthalten von etwa 1 mg bis etwa 500 mg des Wirkstoffs.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen Hilfsstoffen, zu Tabletten oder Dragée-Kernen verarbeiten.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Alginsäure oder ein Salz davon, wie Natriumälginat.

Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol, oder Derivate davon.

Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Oral anwendbare pharmazeutische Zusammensetzungen sind ebenfalls Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten flüssigen Hilfsstoffen, wie fetten Oelen, Paraffinöl oder flüssigen Polyethylenglykolen, gelöst oder suspendiert, wobei ebenfalls Stabilisatoren zugefügt sein können.

Weitere orale Applikationsformen sind z.B. in üblicher Weise bereitete Sirups, die den Wirkstoff z.B. in suspendierter Form und in einer Konzentration von ca. 0,1 % bis 10 %, vorzugsweise ca. 1 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyehylenglykole oder höhere Alkanole.

Zur parenteralen Verabreichung eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Lösungen, wie sie z.B. für die parenterale Verabreichung verwendet werden, können auch als Infusionslösungen angewandt werden.

Die Erfindung betrifft ebenfalls die Verbindungen der Formel I zur Anwendung in einem therapeutischen Verfahren, insbesondere zur Behandlung der oben genannten Krankheitszustände. Die Verbindungen der vorliegenden Erfindung können prophylaktisch oder therapeutisch verabreicht werden, wobei man sie vorzugsweise in Form von pharmazeutischen Präparaten verwendet. Dabei wird bei einem Körpergewicht von etwa 70 kg eine tägliche Dosis von etwa 1 mg bis etwa 1000 mg, vorzugsweise von etwa 25 bis 100 mg oral bzw. 2 bis 50 mg parenteral, einer Verbindung der vorliegenden Erfindung verabreicht.

Die nachfolgenden Beispiele illustrieren die vorliegende Erfindung; Temperaturen werden in Grad Celsius angegeben. Folgende Abkürzungen werden verwendet: DMF = N,N-Dimethylformamid; Ether = Diethylether; Essigester = Essigsäureethylester; THF = Tetrahydrofuran; MS (FAB) = Massenspektrum ("Fast Atom Bombardment").

Beispiel 1: 4-Amidino-1-indanon-2'-amidinohydrazon-dihydrochlorid

Eine Lösung von 3,8 g (27,9 mMol) Aminoguanidin-hydrogencarbonat in 200 ml Wasser und 14,7 ml 2H Salzsäure wird auf 60° erwärmt und unter Rühren innerhalb von 30 min mit einer Lösung von 5,85 g (27,8 mMol) 4-Amidino-1-indanon-hydrochlorid in 200 ml Methanol versetzt. Das Reaktionsgemisch wird 24 h am Rückfluss gekocht und nach dem Abkühlen zur Trockne eingedampft. Der Rückstand wird in 50 ml Ethanol suspendiert, filtriert, mit Ethanol und Ether gewaschen und getrocknet. Man erhält so die Titelverbindung, die 1 Mol Kristallwasser enthält, Smp. >330°; MS(FAB): $(M+H)^+ = 231$; $^1$H-NMR ($D_2O$): $\delta = 8{,}08$ (d,1H); 7,75 (d,1H); 7,58 (t,1H); 3,35 (m, 2H); 2,96 (m,2H).

Die Ausgangsverbindungen werden wie folgt hergestellt:

(a) 4-Thiocarbamoyl-1-indanon

Eine Lösung von 12,1 g (77 mMol) 4-Cyano-1-indanon [Coll. Czechoslov. Chem. Commun. 43, 3227 (1978)] in 220 ml Pyridin und 10,6 ml (77 mMol) Triethylamin wird bei 40° während 3 h mit Schwefelwasserstoff gesättigt und weitere 16 h bei der gleichen Temperatur gerührt. Das Reaktionsgemisch wird nach

dem Abkühlen zur Trockne eingedampft und der Rückstand mit 300 ml Wasser versetzt. Das auskristallisierte gelbe Produkt wird abgesaugt, mit Wasser gewaschen, getrocknet und aus Essigester umkristallisiert. Man erhält so die Ausgangsverbindung (a), Smp. 197° (Zers.).

b) 4-Amidino-1-indanon-hydrochlorid

Eine Lösung von 9,8 g (51,3 mMol) 4-Thiocarbamoyl-1-indanon in 500 ml abs. Methylenchlorid wird bei Raumtemperatur unter Argon mit 10,8 g (54 mMol) Triethyloxoniumtetrafluoroborat versetzt. Nach 16 h gibt man zu der Reaktionslösung ein Gemisch von 4,2 g Kaliumcarbonat und 4,2 ml Wasser. Man rührt kurz nach, filtriert und wäscht das Filtrat mit Wasser. Die organische Phase wird über Magnesiumsulfat getrocknet, filtriert und eingedampft. Der so erhaltene rohe Ethylthio-iminoether wird in 160 ml abs. Ethanol gelöst, mit 3,3 g (60 mMol) Ammoniumchlorid versetzt und 20 h am Rückfluss erhitzt. Nach dem Abkühlen wird das Reaktionsgemisch zur Trockne eingedampft. Die Ausgangsverbindung (b) wird durch Chromatographie an 1000 ml Amberlite® ER-180 Harz (Wasser als Eluiermittel) gereinigt und aus Ethanol/Ether umkristallisiert, Smp. 215-218° (Zers.).

Beispiel 2: 4-Amidino-1-indanon-2'-(N-hydroxyamidino)-hydrazon-dihydrochlorid

Eine Lösung von 316 mg (1,5 mMol) 4-Amidino-1-indanon-hydrochlorid (Beispiel 1b) in 7 ml Methanol wird mit einer Lösung von 394 mg (1,5 mMol) 1-Amino-3-hydroxyguanidin-4-toluolsulfonat in 6 ml Wasser und 0,75 ml (1,5 mMol) 2N Salzsäure versetzt, 2 h am Rückfluss erhitzt und 16 h bei Raumtemperatur gerührt. Das Reaktionsgemisch wird eingedampft und der Rückstand durch Chromatographie (Pharmacia-Säule SR-28-100) an Kieselgel OPTI-UP $C_{12}$ (Wasser als Eluiermittel, 5 ml-Fraktionen, Durchflussgeschwindigkeit 27,5 ml/h) gereinigt. Der Inhalt der Fraktionen 58-78 wird vereinigt, eingedampft und der Rückstand aus Ethanol kristallisiert. Man erhält so die Titelverbindung in Form von wachsartigen Kristallen, MS (FAB): $(M+H)^+$ = 247; $^1$H-NMR ($D_2O$): $\delta$ = 8,06 (d,1H); 7,73 (d,1H); 7,58 (t,1H); 3,36 (m, 2H); 2,98 (m,2H).

Beispiel 3: 5-Amidino-1-tetralon-2'-amidinohydrazon-dihydrochlorid

Eine Lösung von 0,41 g (3 mMol) Aminoguanidin-hydrogencarbonat in 31,5 ml 0,1N Salzsäure wird mit 0,675 g (3 mMol) 5-Amidino-1-tetralon-hydrochlorid versetzt und 72 h am Rückfluss erhitzt. Nach dem Abkühlen dampft man zur Trockne ein und kristallisiert die Titelverbindung aus Methanol/Ether um, Smp. >250°; MS (FAB): $(M+H)^+$ = 245; $^1$H-NMR (DMSO-$d_6$): $\delta$ = 11,3 (s, 1H); 9,5 (m,4H); 8,65 (d,1H); 7,92 (m,3H); 7,52 (d,1H); 7,46 (t,1H); 2,7-2,85 (m,4H); 1,9 (m,4H).

Die Ausgangsverbindungen werden wie folgt hergestellt:

(a) 5-Cyano-1-tetralon

Eine Lösung von von 1,0 g (4,4 mMol) 5-Brom-1-tetralon [J. Org. Chem. 49, 4226 (1984)] in 1,3 ml DMF wird mit 0,41 g (4,5 mMol) Kupfer(I)cyanid versetzt und 6 h bei 160° gerührt. Das Reaktionsgemisch wird danach auf 80° abgekühlt, und man gibt eine Lösung von 1,6 g Eisen(III)chlorid-hexahydrat in 2,5 ml Wasser und 0,44 ml konz. Salzsäure zu. Man rührt 45 min nach, kühlt ab, verdünnt das Reaktionsgemisch mit Wasser und extrahiert mit Toluol. Die organische Phase wird mit Wasser gewaschen, über Magnesiumsulfat getrocknet, filtriert und eingedampft. Man erhält so die Ausgangsverbindung (a) als gelb-orange Kristalle, IR ($CH_2Cl_2$): 2220, 1690 cm$^{-1}$; $^1$H-NMR ($CDCl_3$): $\delta$ = 8,26 (q,1H); 7,81 (q,1H); 7,43 (t,1H); 3,21 (t,2H); 2,72 (t,2H); 2,23 (m,2H).

(b) 5-Thiocarbamoyl-1-tetralon

Analog Beispiel 1a werden 10,6 g (62 mMol) 5-Cyano-1-tetralon in 200 ml Pyridin und 8,6 ml Triethylamin mit Schwefelwasserstoff behandelt und aufgearbeitet. Man erhält so die Ausgangsverbindung (b) als gelbe Kristalle, Smp. 200-205°.

(c) 5-Amidino-1-tetralon-hydrochlorid

Analog Beispiel 1b werden 8,6 g (42 mMol) 5-Thiocarbamoyl-1-tetralon mit 8,8 g (44 mMol) Triethyloxonium-tetrafluoroborat und 2,6 g (49 mMol) Ammoniumchlorid behandelt. Man erhält so die Ausgangsverbindung (c) als leicht rosa gefärbte Kristalle, MS (FAB): $(M+H)^+$ = 189.

Beispiel 4: 4-Thiocarbamoyl-1-indanon-2'-amidinohydrazon-hydrochlorid

Eine Lösung von 1,9 g (10 mMol) 4-Thiocarbamoyl-1-indanon (Beispiel 1a) in 50 ml Ethanol wird mit 1,36 g (10 mMol) Aminoguanidin-hydrogencarbonat und 10 ml 2N Salzsäure versetzt und 24 h am Rückfluss erhitzt. Das Reaktionsgemisch wird nach dem Abkühlen zur Trockne eingedampft, wobei man die Titelverbindung erhält.

Beispiel 5: 4-Amidino-1-indanon-2'-amidinohydrazon-dihydrochlorid

Analog Beispiel 1b wird 4-Thiocarbamoyl-1-indanon-2'-amidinohydrazon-hydrochlorid (Beispiel 4) mit Triethyloxonium-tetrafluoroborat und Ammoniumchlorid umgesetzt, wobei man die Titelverbindung erhält, Smp. >330°; MS (FAB): $(M+H)^+$ = 231; $^1$1H-NMR ($D_2O$): $\delta = 8{,}08$ (d,1H); 7,75 (d,1H); 7,58 (t,1H); 3,35 (m,2H); 2,96 (m,2H).

Beispiel 6: 4-Amidino-1-indanon-2'-amidinohydrazon-dihydrochlorid

Eine Lösung von 0,26 g (1 mMol) 4-Cyano-1-indanon-2'-amidinohydrazon-hydrochlorid in 5 ml abs. Methanol wird mit 1,2 ml einer 1N Natriummethoxid-Lösung in Methanol versetzt und 16 h am Rückfluss erhitzt. Nach dem Abkühlen gibt man zum Reaktionsgemisch 0,16 g (3 mMol) festes Ammoniumchlorid zu und rührt 1 h bei 60°. Das Reaktionsgemisch wird danach eingedampft und der Rückstand aus verdünntem Ethanol kristallisiert. Man erhält so die Titelverbindung, Smp. >330°.

Die Ausgangsverbindung wird wie folgt hergestellt:

(a) 4-Cyano-1-indanon-2'-amidinohydrazon-hydrochlorid

Analog Beispiel 1 werden 314 mg (2 mMol) 4-Cyano-1-indanonin 20 ml Methanol gelöst, mit einer Lösung von 272 mg (2 mMol) Aminoguanidin-hydrogencarbonat in 9 ml Wasser und 1 ml 2N Salzsäure versetzt und 4 Tage am Rückfluss gerührt. Nach dem Abkühlen wird das Reaktionsgemisch zur Trockne eingedampft und der Rückstand aus Wasser kristallisiert. Man erhält so die Ausgangsverbindung (a), Smp. >230°; $^1$H-NMR ($DMSO\text{-}d_6/D_2O$): $\delta = 8{,}16$ (d,1H); 7,9 (d,1H); 7,55 (t,1H); 3,28 (m,2H); 2,9 (m,2H); IR(Nujol): 2190 $cm^{-1}$ (CN).

Beispiel 7: 4-(N-Hydroxyamidino)-1-indanon-2'-amidinohydrazon-dihydrochlorid

0,2 g (3 mMol) Hydroxylamin-hydrochlorid werden in 1 ml abs. Ethanol suspendiert und mit 2 ml einer 1N Natrium-ethoxid-Lösung in Ethanol versetzt. Dieses Gemisch wird 1 h gerührt und filtriert. Zum Filtrat wird eine Lösung von 0,26 g (1 mMol) 4-Cyano-1-indanon-2'-amidinohydrazon-hydrochlorid (Beispiel 6a) in 2 ml Wasser gegeben, und man erhitzt 6 h am Rückfluss. Nach dem Abkühlen wird das Reaktionsgemisch eingedampft und die Titelverbindung aus Wasser kristallisiert, Smp. >250°; $^1$H-NMR ($DMSO\text{-}d_6 + D_2O$): $\delta$ = 8,12 (m,1H); 7,55 (m,2H); 3,22 (m,2H); 2,83 (m,2H).

Beispiel 8: 4-Amidino-2-methyl-1-indanon-2'-amidinohydrazon-dihydrochlorid

Analog Beispiel 1 wird ausgehend von 4-Cyano-2-methyl-1-indanon (s. US-Patent 3 956 363) die Titelverbindung hergestellt.

Beispiel 9: 5-Amidino-6-methoxy-1-tetralon-2'-amidinohydrazon-dihydrochlorid

Analog Beispiel 1 wird ausgehend von 5-Cyano-6-methoxy-1-tetralon [Chem. Pharm. Bull. 31, 2329 (1983)] die Titelverbindung hergestellt.

Beispiel 10: 4-Amidino-6-methyl-1-indanon-2'-amidinohydrazon-dihydrochlorid

Analog Beispiel 3 wird ausgehend von 4-Brom-6-methyl-1-indanon (Bull. Soc. Chim. France 1964, 3103) die Titelverbindung hergestellt.

Beispiel 11: 4-Amidino-6-methoxy-7-methyl-1-indanon-2'-amidinohydrazon-dihydrochlorid

Analog Beispiel 3 wird ausgehend von 4-Brom-6-methoxy-7-methyl-1-indanon (J. Chem. Soc. Perkin Trans. 1 1974, 1911) die Titelverbindung hergestellt.

Beispiel 12: 4-Amidino-6,7-dimethyl-1-indanon-2'-amidinohydrazon-dihydrochlorid

Analog Beispiel 3 wird ausgehend von 4-Brom-6, 7-dimethyl-1-indanon [J. Het. Chem. 24, 677 (1987)] die Titelverbindung hergestellt.

Beispiel 13: 4-Amidino-7-hydroxy-3-methyl-1-indanon-2'-amidinohydrazon-dihydrochlorid

Analog Beispiel 3 wird ausgehend von 4-Brom-7-hydroxy-3-methyl-1-indanon [Indian J. Chem. Sect. B 24B, 1061 (1985)] die Titelverbindung hergestellt.

Beispiel 14: 4-(Methylamidino)-1-indanon-2'-amidinohydrazon-dihydrochlorid

Analog Beispiel 1b wird 4-Thiocarbarnoyl-1-indanon-2'-amidinohydrazon-hydrochlorid (Beispiel 4) mit Triethyloxonium-tetrafluoroborat und Methylammoniumchlorid umgesetzt, wobei man die Titelverbindung erhält.

Beispiel 15: 4-Amidino-1-indanon-2'-amidinohydrazon-dihydrochlorid

Eine Lösung von 6,12 g (45 mMol) Aminoguanidin-hydrogencarbonat in 100 ml Wasser und 46 ml 1N Salzsäure wird mit 9,45 g (44,9 mMol) 4-Amidino-1-indanon-hydrochlorid (s. Beispiel 1b) versetzt und 24 h bei 24° gerührt. Das auskristallisierte Produkt wird abgesaugt, mit wenig Wasser gewaschen und aus 300 ml Wasser umkristallisiert. Man erhält so die Titelverbindung, die 1 Mol Kristallwasser enthält, Smp. >330°; MS (FAB): $(M + H)^+ = 231$; $^1$H-NMR ($D_2O$): $\delta$ = 8,08 (d,1H); 7,75 (d,1H); 7,58 (t,1H); 3,35 (m,2H); 2,96 (m,2H).

Beispiel 16: 4-Amidino-2-methyl-1-indanon-2'-amidinohydrazon-dihydrochlorid

Eine Lösung von 1,0 g (5,0 mMol) 4-Amidino-2-methyl-1-indanon-hydrochlorid und 0,68 g (5,0 mMol) Aminoguanidin-hydrogencarbonat in 10 ml 0,5N Salzsäure wird 120 h bei 25° gerührt. Das auskristallisierte Produkt wird abgesaugt, mit wenig Wasser gewaschen und getrocknet. Man erhält so die Titelverbindung, Smp. >250°; MS (FAB): $(M + H)^+ = 245$; $^1$H-NMR ($D_2O$): $\delta$ = 7,95 (d,1H); 7,66 (d,1H); 7,48 (t,1H); 3,45 (m,2H); 2,85 (d,1H); 1,12 (d,3H).

Die Ausgangsverbindungen werden wie folgt hergestellt:

(a) 4-Thiocarbamoyl-2-methyl-1-indanon

Analog Beispiel 1a werden 11,1 g (65 mMol) 4-Cyano-2-methyl-1-indanon [s. US-Patent 3 956 363] in 200 ml Pyridin und 9,7 ml Triethylamin mit Schwefelwasserstoff behandelt und aufgearbeitet. Man erhält so die Ausgangsverbindung (a) als gelbe Kristalle, Smp. 195-198° (Zers.); $^1$H-NMR (DMSO-$d_6$): $\delta$ = 9,61 (s,1H); 7,71 (m,2H); 7,48 (m,1H); 3,48 (m,1H); 2,81 (m,2H); 1,23 (s,3H); 1,19 (s,3H).

(b) 4-Amidino-2-methyl-1-indanon-hydrochlorid

Analog Beispiel 1b werden 10,2 g (50 mMol) der Ausgangsverbindung (a) mit 11,0 g (55 mMol) Triethyloxonium-tetrafluoroborat und 3,2 g (60 mMol) Ammoniumchlorid behandelt. Man erhält so die Ausgangsverbindung (b) als rosa Kristalle. Sie wird direkt weiter umgesetzt.

Beispiel 17: 4-Amidino-6,7-dimethyl-1-indanon-2'-amidinohydrazon-dihydrochlorid

Analog Beispiel 1 wird ausgehend von 4-Amidino-6,7-dimethyl-1-indanon-hydrochlorid die Titelverbindung hergestellt, Smp. >240°C; MS (FAB): $(M+H)^{\oplus} = 259$; $^1$H-NMR ($D_2O$): $\delta$ = 7,43 (s,1H); 3,12 (m,2H); 2,75 (m,2H); 2,43 (s,3H); 2,24 (s,3H).

Die Ausgangsverbindungen werden wie folgt hergestellt:

(a) 4-Cyano-6,7-dimethyl-1-indanon

Ein Gemisch von 17,8 g (74,5 mMol) 4-Brom-6,7-dimethyl-1-indanon [J. Het. Chem. 24, 677 (1987)] und 7,3 g (82 mMol) Kupfer(I)cyanid in 18 ml DMF wird 6 h bei 170° gerührt. Das Reaktionsgemisch wird danach auf 100° abgekühlt und nacheinander mit 200 ml Toluol und einer Lösung von 31,2 g Eisen(III)-chlorid-hexahydrat in 47 ml Wasser und 8,2 ml konz. Salzsäure versetzt. Man rührt 20 min bei 70° nach, kühlt ab und verdünnt das Reaktionsgemisch mit Toluol und Wasser. Die organische Phase wird abgetrennt, mit Wasser, einer halbgesättigten Natriumhydrogencarbonatlösung und wieder mit Wasser gewaschen, getrocknet und eingedampft. Der Rückstand wird aus Essigester und Ether kristallisiert und entspricht der Ausgangsverbindung (a). Man erhält beige Kristalle vom Smp. 160-163°; IR ($CH_2Cl_2$): 2220, 1710 $cm^{-1}$.

(b) 4-Thiocarbamoyl-6,7-dimethyl-1-indanon

Analog Beispiel 1a werden 10 g (54,1 mMol) der Ausgangsverbindung (a) in 200 ml Pyridin und 7,5 ml Triethylamin mit Schwefelwasserstoff behandelt und aufgearbeitet. Man erhält so die Ausgangsverbindung (b) in Form von gelben Kristallen, Smp. 207-208°; $^1$H-NMR (DMSO-$d_6$): $\delta$ = 10,03 (s,1H); 9,49 (s,1H); 7,49 (s,1H); 3,12 (m,2H); 2,61 (m,2H); 2,54 (s,3H); 2,29 (s,3H).

(c) 4-Amidino-6,7-dimethyl-1-indanon-hydrochlorid

Analog Beispiel 1b werden 4,4 g (20 mMol) der Ausgangsverbindung (b) mit 4,26 g (21 mMol) Triethyloxonium-tetrafluoroborat und 1,2 g (24 mMol) Ammoniumchlorid behandelt. Man erhält so die Ausgangsverbindung (c) in Form von beigen Kristallen.

Beispiel 18: 4-Amidino-6,7-dimethoxy-1-indanon-2'-amidinohydrazon-dihydrochlorid

Eine Lösung von 0,4 g (3 mMol) Aminoguanidin-hydrogencarbonat in 6 ml 0,5 N Salzsäure wird mit 0,73 g (2,7 mMol) 4-Amidino-6,7-dimethoxy-1-indanon-hydrochloridversetzt und 24 h bei 50° gerührt. Mach dem Abkühlen wird das auskristallisierte Produkt abgesaugt, mit wenig Wasser gewaschen und getrocknet, wobei man die Titelverbindung erhält, Smp. >220°; MS (FAB): $(M + H)^+$ = 291; $^1$H-NMR ($D_2O$): $\delta$ = 7,45 (s,1H); 3,97 (s,6H); 3,27 (m,2H); 2,98 (m,2H).

Die Ausgangsverbindungen werden wie folgt hergestellt:

(a) 4-Cyano-6,7-dimethoxy-1-indanon

Ein Gemisch von 6,57 g (24,2 mMol) 4-Brom-6,7-dimethoxy-1-indanon [Can. J. Chem. 57, 1603 (1979)] und 2,5 g (28 mMol) Kupfer(I)cyanid in 7 ml DMF wird 5,75 h bei 170° gerührt. Das Reaktionsgemisch wird danach auf 100° abgekühlt und nacheinander mit 70 ml Toluol und einer Lösung von 9,7 g (36 mMol) Eisen(III)chlorid-hexahydrat in 15,6 ml Wasser und 3,5 ml konz. Salzsäure versetzt. Man rührt 30 min bei 80° nach, kühlt ab und verdünnt das Reaktionsgemisch mit Toluol und Wasser. Die organische Phase wird abgetrennt, mit Wasser, einer halbgesättigten Natriumhydrogencarbonatlösung und wieder mit Wasser gewaschen, getrocknet und eingedampft. Der Rückstand wird bei 150-160°/0,1 mbar in einer Kugelrohrdestille destilliert und entspricht der Ausgangsverbindung (a), Smp. 150°; IR ($CH_2Cl_2$): 2220, 1710 $cm^{-1}$; $^1$H-NMR ($CDCl_3$): $\delta$ = 7,33 (s,1H); 4,12 (s,3H); 3,90 (s,3H); 3,19 (m,2H); 2,76 (m,2H).

(b) 4-Thiocarbamoyl-6,7-dimethoxy-1-indanon

Analog Beispiel 1a werden 3,7 g (17 mMol) der Ausgangsverbindung (a) in 100 ml Pyridin und 2,4 ml Triethylamin mit Schwefelwasserstoff behandelt und aufgearbeitet. Man erhält so die Ausgangsverbindung (b) in Form von hellgelben Kristallen, Smp. 196-199°; $^1$H-NMR (DMSO-$d_6$): $\delta$ = 10,06 (s,1H); 9,50 (s,1H); 7,41 (s,1H); 3,84 (s,6H); 3,13 (m,2H); 2,63 (m,2H).

(c) 4-Amidino-6,7-dimethoxy-1-indanon-hydrochlorid

Analog Beispiel 1b werden 3,3 g (13 mMol) der Ausgangsverbindung (b) mit 2,8 g (14 mMol) Triethyloxonium-tetrafluoroborat und 0,8 g (15 mMol) Ammoniumchlorid behandelt. Man erhält so die Ausgangsverbindung (c) in Form von beigen Kristallen, Smp. 188° (mit Zers.); $^1$H-NMR (DMSO-$d_6$): $\delta$ = 9,4

(s,3H); 7,63 (s,1H); 3,92 (s,3H); 3,89 (s,3H); 3,18 (m,2H); 2,68 (m,2H).

Beispiel 19: 4-Amidino-3-methyl-1-indanon-2'-amidinohydrazon-dihydrochlorid

Eine Lösung von 300 mg (1,3 mMol) 4-Amidino-3-methyl-1-indanon-hydrochlorid in 6 ml Wasser wird mit 300 mg (2,3 mMol) Aminoguanidin-hydrogencarbonat in 4 ml 0,5N Salzsäure versetzt und 24 h bei 80° gerührt. Das Reaktionsgemisch wird abgekühlt, eingedampft und der Rückstand durch Chromatographie an 180 ml Amberlite® ER-180-Harz mit Wasser als Eluiermittel gereinigt. Die Titelverbindung wird aus Methanol/Ether umkristallisiert, Smp. >250°; $R_f$ = 0,18 (Kieselgel, Methylenchlorid/Methanol/konz. Ammoniak 5:3:1); MS (FAB): $(M + H)^+$ = 245; $^1$H-NMR ($D_2O$): δ = 7,97 (d,1H); 7,64 (d,1H); 7,49 (t,1H); 3,86 (m,1H); 3,17 (q,1H); 2,49 (d,1H); 1,24 (d,3H).

Die Ausgangsverbindungen werden wie folgt hergestellt:

(a) 4-Cyano-3-methyl-1-indanon

Ein Gemisch von 2,6 g (11,5 mMol) 4-Brom-3-methyl-1-indanon [J. Org. Chem. 22, 1019 (1957] und 1,14 g (12,7 mMol) Kupfer(I)cyanid in 2,5 ml DMF wird 6 h bei 170° gerührt. Das Reaktionsgemisch wird danach auf 100° abgekühlt und nacheinander mit 50 ml Toluol und einer Lösung von 4,5 g (16,5 mMol) Eisen(III)chlorid-hexahydrat in 7 ml Wasser und 1,7 ml konz. Salzsäure versetzt. Man rührt 20 min bei 70° nach, kühlt ab und verdünnt das Reaktionsgemisch mit Toluol und Wasser. Die organische Phase wird abgetrennt, mit Wasser, einer halbgesättigten Natriumhydrogencarbonatlösung und wieder mit Wasser gewaschen, getrocknet und eingedampft. Der Rückstand wird bei 100-120°/0,05 mbar in einer Kugelrohrdestille destilliert und entspricht der Ausgangsverbindung (a), Smp. 109-111°; IR ($CH_2Cl_2$): 2220, 1710 $cm^{-1}$; $^1$H-NMR ($CDCl_3$): δ = 7,92 (m,2H); 7,52 (t,1H); 3,73 (m,1H); 3,03 (q,1H); 2,40 (q,1H); 1,55 (d,3H).

(b) 4-Thiocarbamoyl-3-methyl-1-indanon

Analog Beispiel 1a werden 1,45 g (8,47 mMol) der Ausgangsverbindung (a) in 25 ml Pyridin und 1,2 ml Triethylamin mit Schwefelwasserstoff behandelt und aufgearbeitet. Man erhält so die Ausgangsverbindung (b) in Form von blassgelben Kristallen, Smp. 198-200°; $^1$H-NMR (DMSO-$d_6$): δ = 9,78 (s,1H); 7,65 (m,2H); 7,46 (m,1H); 3,98 (m,1H); 2,95 (q,1H); 2,26 (q,1H); 1,25 (d,3H).

(c) 4-Amidino-3-methyl-1-indanon-hydrochlorid

Analog Beispiel 1b werden 0,96 g (4,68 mMol) der Ausgangsverbindung (b) mit 1,0 g (4,93 mMol) Triethyloxonium-tetrafluoroborat und 0,3 g (6 mMol) Ammoniumchlorid behandelt. Man erhält so die Ausgangsverbindung (c) in Form von beigen Kristallen, $^1$H-NMR (DMSO-$d_6$): δ = 9,59 (s,4H); 7,65 (m,2H); 7,46 (m,1H); 3,98 (m,1H); 2,95 (q,1H); 2,26 (q,1H); 1,25 (d,3H).

Beispiel 20: 4-Amidino-1-indanon-2'-amidinohydrazon-dihydrochlorid

Ein Gemisch von 0,32 g (1 mMol) 4-(N-Hydroxyamidino)-1-indanon-2'-amidinohydrazon-dihydrochlorid (Beispiel 7), 0,36 ml (2 mMol) Triethylamin und 0,2 g (1 mMol Eisenpentacarbonyl in 10 ml abs. THF wird 16 h am Rückfluss gekocht. Das Reaktionsgemisch wird danach eingedampft und der Rückstand aus verdünnter Salzsäure kristallisiert, wobei man die Titelverbindung erhält, Smp. >330°.

Beispiel 21: 4-Amidino-2-ethyl-1-indanon-2'-amidinohydrazon-dihydrochlorid

3-(2-Bromphenyl)-2-ethyl-propionsäure (Deutsches Patent 2 733 868) wird in der Wärme mit Polyphosphorsäure zum entsprechenden 1-Indanon cyclisiert und analog Beispiel 3 in die Titelverbindung umgewandelt.

Beispiel 22: 4-Amidino-2-n-butyl-1-indanon-2'-amidinohydrazon-dihydrochlorid

3-(2-Bromphenyl)-2-n-butyl-propionsäure (Deutsches Patent 2 733 868) wird in der Wärme mit Polyphosphorsäure zum entsprechenden 1-Indanon cyclisiert und analog Beispiel 3 in die Titelverbindung umgewandelt.

Beispiel 23: Kapseln enthaltend 0,25 g Wirkstoff, z.B. von einer der Verbindungen der Beispiele 1-22, können wie folgt hergestellt werden:

Zusammensetzung (für 5000 Kapseln)

| | |
|---|---|
| Wirkstoff | 1250 g |
| Talk | 180 g |
| Weizenstärke | 120 g |
| Magnesiumstearat | 80 g |
| Laktose | 20 g |

Die pulverförmigen Substanzen werden durch ein Sieb mit einer Maschenweite von 0,6 mm getrieben und gemischt. Portionen von je 0,33 g des Gemisches werden mittels einer Kapselfüllmaschine in Gelatine-Kapseln abgefüllt.

Beispiel 24: Es werden 10000 Tabletten hergestellt, die je 5 mg Wirkstoff, z.B. eine der in den Beispielen 1-22 hergestellten Verbindungen, enthalten:

Zusammensetzung:

| | |
|---|---|
| Wirkstoff | 50,00 g |
| Milchzucker | 2535,00 g |
| Maisstärke | 125,00 g |
| Polyethylenglykol 6.000 | 150,00 g |
| Magnesiumstearat | 40,00 g |
| Gereinigtes Wasser | quantum satis |

Verfahren: Alle pulverigen Bestandteile werden durch ein Sieb mit einer Maschenweite von 0,6 mm gesiebt. Dann wird der Wirkstoff, der Milchzucker, das Magnesiumstearat und die Hälfte der Stärke in einem geeigneten Mischer vermischt. Die andere Hälfte der Stärke wird in 65 ml Wasser suspendiert und die entstandene Suspension zu einer siedenden Lösung des Polyethylenglykols in 260 ml Wasser gegeben. Die gebildete Paste wird dem Pulvergemisch zugesetzt und gegebenenfalls unter Zugabe von mehr Wasser granuliert. Das Granulat wird über Macht bei 35 °C getrocknet, durch ein Sieb mit 1,2 mm Maschenweite getrieben und zu Tabletten, welche eine Bruchrille aufweisen, gepresst.

## Patentansprüche

## Patentansprüche für folgende Vertagsstaaten : AT, BE, CH, DE, DK, GB, FR, IT, LI, LU, NL, SE

**1.** Verbindungen der Formel I

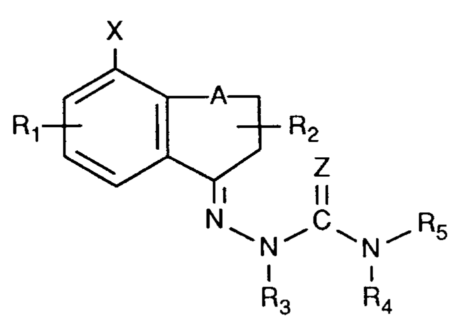

(I)

worin A eine direkte Bindung oder $-(CH_2)_n-$, worin n für 1, 2 oder 3 steht, bedeutet; X für einen Rest $-C(=Y)-NR_6R_7$ steht; Y für $NR_8$, O oder S steht; Z für $NR_9$, O oder S steht; $R_1$ und $R_2$ unabhängig voneinander Wasserstoff oder einen oder mehrere Substituenten ausgewählt aus Niederalkyl, Trifluormethyl, $C_3$-$C_8$-Cycloalkyl, welches unsubstituiert oder durch Niederalkyl substituiert ist, Arylniederalkyl,

Hydroxy, Niederalkoxy, Arylniederalkoxy, Aryloxy, Niederalkanoyloxy, Halogen, Amino, N-Niederalkylamino, N,N-Diniederalkylamino, Niederalkanoylamino, Nitro, Niederalkanoyl, Arylcarbonyl, Carboxy, Niederalkoxycarbonyl, Carbamoyl, N-Niederalkylcarbamoyl, N,N-Diniederalkylcarbamoyl, N-Arylcarbamoyl, Cyano, Mercapto, Niederalkylthio, Niederalkylsulfonyl, Sulfamoyl, N-Niederalkylsulfamoyl und N-N-Diniederalkylsulfamoyl bedeuten; die Reste $R_3$, $R_4$, $R_6$, $R_8$ und $R_9$ unabhängig voneinander Wasserstoff oder Niederalkyl bedeuten; und $R_5$ und $R_7$ unabhängig voneinander für Wasserstoff, Niederalkyl, Hydroxy, Niederalkoxy, Niederalkanoyloxy, unsubstituiertes Amino, Diniederalkylamino, $C_4$-$C_7$-Alkylenamino oder Oxa-, Thia- oder Aza-$C_4$-$C_7$-alkylenamino stehen; Tautomere davon, oder Salze davon; wobei Aryl für Phenyl oder Naphthyl steht, welches jeweils unsubstituiert oder durch einen oder mehrere Substituenten ausgewählt aus Niederalkyl, Niederalkoxy, Hydroxy, Niederalkanoyloxy, Nitro, Amino, Halogen, Trifluormethyl, Carboxy, Niederalkoxycarbonyl, Carbamoyl, N-Niederalkylcarbamoyl, N,N-Diniederalkylcarbamoyl, Cyano, Niederalkanoyl, Arylcarbonyl, Niederalkylsulfonyl, Sulfamoyl, N-Niederalkylsulfamoyl und N,N-Diniederalkylsulfamoyl substituiert ist; und wobei "Nieder" oder "nieder" einen Rest bis und mit maximal 7 Kohlenstoffatomen bezeichnet.

**2.** Verbindungen der Formel I gemäss Anspruch 1, worin A eine direkte Bindung oder $-(CH_2)_n-$, worin n für 1 oder 2 steht, bedeutet; X für einen Rest $-C(=Y)NR_6R_7$ steht; Y für $NR_8$, O oder S steht; Z für $NR_9$, O oder S steht; $R_1$ und $R_2$ unabhängig voneinander Wasserstoff oder einen oder zwei Substituenten aus der Gruppe bestehend aus Niederalkyl, Trifluormethyl, Cycloalkyl, Arylniederalkyl, Hydroxy, Niederalkoxy, Arylniederalkoxy, Aryloxy, Niederalkanoyloxy, Halogen, Amino, N-Niederalkylamino, N,N-Diniederalkylamino, Niederalkanoylamino, Nitro, Niederalkanoyl, Arylcarbonyl, Carboxy, Niederalkoxycarbonyl, Carbamoyl, N-Niederalkylcarbamoyl, N,N-Diniederalkylcarbamoyl, N-Arylcarbamoyl, Cyano, Mercapto, Niederalkylthio, Niederalkylsulfonyl, Sulfamoyl, N-Niederalkylsulfamoyl und N,N-Diniederalkylsulfamoyl bedeuten, wobei Aryl für Phenyl steht, das unsubstituiert oder durch Niederalkyl, Niederalkoxy, Hydroxy, Halogen oder Trifluormethyl substituiert ist; die Reste $R_3$, $R_4$, $R_6$, $R_8$ und $R_9$ unabhängig voneinander Wasserstoff oder Niederalkyl bedeuten; und $R_5$ und $R_7$ unabhängig voneinander für Wasserstoff, Niederalkyl, Hydroxy, Niederalkoxy, Niederalkanoyloxy, Amino, Niederalkylamino, Diniederalkylamino, $C_4$-$C_7$-Alkylenamino oder Oxa-, Thia- oder Aza-$C_4$-$C_7$-alkylenamino stehen; Tautomere davon, oder Salze davon; wobei "Nieder" oder "nieder" einen Rest bis und mit maximal 7 Kohlenstoffatomen bezeichnet.

**3.** Verbindungen der Formel I gemäss Anspruch 1, worin A eine direkte Bindung oder $-(CH_2)_n-$, worin n für 1 oder 2 steht, bedeutet; X für einen Rest $-C(=Y)-NR_6R_7$ steht; Y für NH, O oder S steht; Z für NH, O oder S steht; $R_1$ und $R_2$ unabhängig voneinander Wasserstoff oder einen oder zwei Substituenten aus der Gruppe bestehend aus Niederalkyl, Trifluormethyl, Phenylniederalkyl, Hydroxy, Niederalkoxy und Halogen bedeuten; die Reste $R_3$, $R_4$ und $R_6$ Wasserstoff bedeuten; und $R_5$ und $R_7$ unabhängig voneinander für Wasserstoff, Niederalkyl, Hydroxy oder Amino stehen; Tautomere davon, oder Salze davon; wobei "Nieder" oder "nieder" einen Rest bis und mit maximal 7 Kohlenstoffatomen bezeichnet.

**4.** Verbindung der Formel I gemäss Anspruch 1, worin A eine direkte Bindung oder $-CH_2$-bedeutet; X für einen Rest $-C(=Y)-NR_6R_7$ steht; Y für NH oder S steht; Z für NH steht; $R_1$ Wasserstoff oder einen oder zwei Substituenten aus der Gruppe bestehend aus Niederalkyl, Hydroxy, Niederalkoxy und Halogen bedeutet; $R_2$ für Wasserstoff oder Niederalkyl steht; die Reste $R_3$, $R_4$ und $R_6$ Wasserstoff bedeuten; und $R_5$ und $R_7$ unabhängig voneinander für Wasserstoff, Niederalkyl oder Hydroxy stehen; Tautomere davon, oder pharmazeutisch anwendbare Salze davon; wobei "Nieder" oder "nieder" einen Rest bis und mit maximal 7 Kohlenstoffatomen bezeichnet.

**5.** Verbindungen der Formel I gemäss Anspruch 1, worin A eine direkte Bindung bedeutet, X für einen Rest $-C(=NH)-NH_2$ steht; Z für NH steht; $R_1$ Wasserstoff oder einen oder zwei Substituenten aus der Gruppe bestehend aus Niederalkyl, Hydroxy und Niederalkoxy bedeutet; die Reste $R_2$, $R_3$ und $R_4$ Wasserstoff bedeuten; und $R_5$ für Wasserstoff oder Hydroxy steht; Tautomere davon, oder pharmazeutisch anwendbare Salze davon; wobei "Nieder" oder "nieder" einen Rest bis und mit maximal 7 Kohlenstoffatomen bezeichnet.

**6.** 4-Amidino-1-indanon-2'-amidinohydrazon gemäss Anspruch 1, oder ein pharmazeutisch anwendbares Salz davon.

7. 4-Amidino-1-indanon-2'-(N-hydroxyamidino)-hydrazon gemäss Anspruch 1, oder ein pharmazeutisch anwendbares Salz davon.

8. 5-Amidino-1-tetralon-2'-amidinohydrazon gemäss Anspruch 1, oder ein pharmazeutisch anwendbares Salz davon.

9. Pharmazeutische Präparate enthaltend eine Verbindung gemäss einem der Ansprüche 1 bis 8 und mindestens ein pharmazeutisch verwendbares Trägermaterial.

10. Eine Verbindung gemäss einem der Ansprüche 1 bis 8 zur Anwendung in einem Verfahren zur therapeutischen Behandlung des tierischen oder menschlichen Körpers.

11. Eine Verbindung gemäss einem der Ansprüche 1 bis 8 zur Verwendung bei der Behandlung von Krankheiten, die auf eine Hemmung des Enzyms S-Adenosylmethionindecarboxylase ansprechen.

12. Verwendung einer Verbindung gemäss einem der Ansprüche 1 bis 8 zur Herstellung pharmazeutischer Präparate.

13. Verwendung einer Verbindung gemäss einem der Ansprüche 1 bis 8 zur Herstellung pharmazeutischer Präparate für die Behandlung von Krankheiten, die auf eine Hemmung des Enzyms S-Adenosylmethionindecarboxylase ansprechen.

14. Verfahren zur Herstellung einer Verbindung der Formel I gemäss Anspruch 1, dadurch gekennzeichnet, dass man
(a) eine Verbindung der Formel II

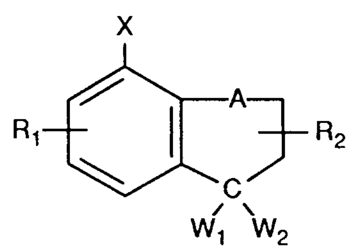

(II)

worin die Gruppe $CW_1W_2$ für Carbonyl, funktionell abgewandeltes Carbonyl oder geschütztes Carbonyl steht und A, X, $R_1$ und $R_2$ die unter Formel I angegebene Bedeutung haben, mit einem Amin der Formel III

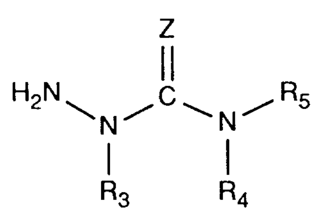

(III)

worin Z, $R_3$, $R_4$ und $R_5$ wie unter Formel I definiert sind, kondensiert, oder

(b) in einer Verbindung der Formel IV

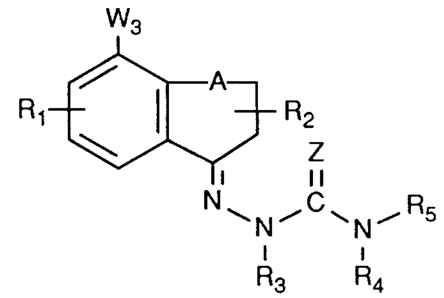

(IV)

worin $W_3$ einen Rest, der in eine Gruppe X der Formel I überführt werden kann, bedeutet und A, Z, $R_1$, $R_2$, $R_3$, $R_4$ und $R_5$ wie unter Formel I definiert sind, den Rest $W_3$ in die Gruppe X überführt; und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt.

**Patentansprüche für folgende Vertragsstaaten : ES, GR**

**1.** Verfahren zur Herstellung von Verbindungen der Formel I

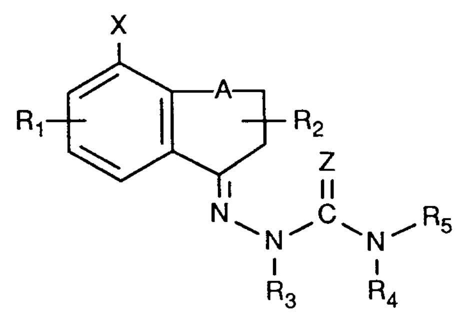

(I)

worin A eine direkte Bindung oder $-(CH_2)_n-$, worin n für 1, 2 oder 3 steht, bedeutet; X für einen Rest $-C(=Y)-NR_6R_7$ steht; Y für $NR_8$, O oder S steht; Z für $NR_9$, O oder S steht; $R_1$ und $R_2$ unabhängig voneinander Wasserstoff oder einen oder mehrere Substituenten ausgewählt aus Niederalkyl, Trifluormethyl, $C_3$-$C_8$-Cycloalkyl, welches unsubstituiert oder durch Niederalkyl substituiert ist, Arylniederalkyl, Hydroxy, Niederalkoxy, Arylniederalkoxy, Aryloxy, Niederalkanoyloxy, Halogen, Amino, N-Niederalkylamino, N,N-Diniederalkylamino, Niederalkanoylamino, Nitro, Niederalkanoyl, Arylcarbonyl, Carboxy, Niederalkoxycarbonyl, Carbamoyl, N-Niederalkylcarbamoyl, N,N-Diniederalkylcarbamoyl, N-Arylcarbamoyl, Cyano, Mercapto, Niederalkylthio, Niederalkylsulfonyl, Sulfamoyl, N-Niederalkylsulfamoyl und N-N-Diniederalkylsulfamoyl bedeuten; die Reste $R_3$, $R_4$, $R_6$, $R_8$ und $R_9$ unabhängig voneinander Wasserstoff oder Niederalkyl bedeuten; und $R_5$ und $R_7$ unabhängig voneinander für Wasserstoff, Niederalkyl, Hydroxy, Niederalkoxy, Niederalkanoyloxy, unsubstituiertes Amino, Diniederalkylamino, $C_4$-$C_7$-Alkylenamino oder Oxa-, Thia- oder Aza-$C_4$-$C_7$-alkylenamino stehen; Tautomeren davon, oder Salzen davon; wobei Aryl für Phenyl oder Naphthyl steht, welches jeweils unsubstituiert oder durch einen oder mehrere Substituenten ausgewählt aus Niederalkyl, Niederalkoxy, Hydroxy, Niederalkanoyloxy, Nitro, Amino, Halogen, Trifluormethyl, Carboxy, Niederalkoxycarbonyl, Carbamoyl, N-Niederalkylcarbamoyl, N,N-Diniederalkylcarbamoyl, Cyano, Niederalkanoyl, Arylcarbonyl, Niederalkylsulfonyl, Sulfamoyl, N-Niederalkylsulfamoyl und N,N-Diniederalkylsulfamoyl substituiert ist; und wobei "Nieder" oder "nieder" einen Rest bis und mit maximal 7 Kohlenstoffatomen bezeichnet; dadurch gekennzeichnet, dass man

(a) eine Verbindung der Formel II

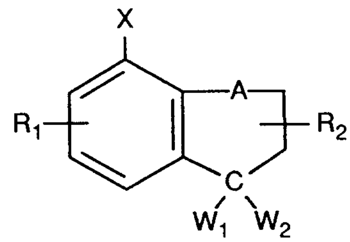

(II)

worin die Gruppe $CW_1W_2$ für Carbonyl, funktionell abgewandeltes Carbonyl oder geschütztes Carbonyl steht und A, X, $R_1$ und $R_2$ die unter Formel I angegebene Bedeutung haben, mit einem Amin der Formel III

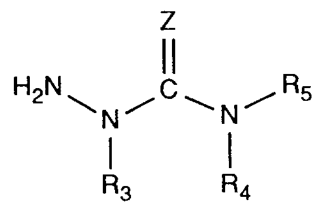

(III)

worin Z, $R_3$, $R_4$ und $R_5$ wie unter Formel I definiert sind, kondensiert, oder
(b) in einer Verbindung der Formel IV

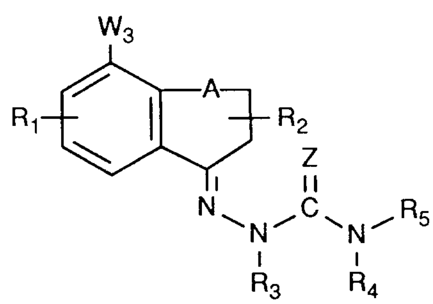

(IV)

worin $W_3$ einen Rest, der in eine Gruppe X der Formel I überführt werden kann, bedeutet und A, Z, $R_1$, $R_2$, $R_3$, $R_4$ und $R_5$ wie unter Formel I definiert sind, den Rest $W_3$ in die Gruppe X überführt; und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt.

2. Verfahren gemäss Anspruch 1 zur Herstellung von Verbindungen der Formel I, worin A eine direkte Bindung oder $(CH_2)_n$-, worin n für 1 oder 2 steht, bedeutet; X für einen Rest -C(=Y) $NR_6R_7$ steht; Y für $NR_8$, O oder S steht; Z für $NR_9$, O oder S steht; $R_1$ und $R_2$ unabhängig voneinander Wasserstoff oder einen oder zwei Substituenten aus der Gruppe bestehend aus Niederalkyl, Trifluormethyl, Cycloalkyl, Arylniederalkyl, Hydroxy, Niederalkoxy, Arylniederalkoxy, Aryloxy, Niederalkanoyloxy, Halogen, Amino, N-Niederalkylamino, N,N-Diniederalkylamino, Niederalkanoylamino, Nitro, Niederalkanoyl, Arylcarbonyl, Carboxy, Niederalkoxycarbonyl, Carbamoyl, N-Niederalkylcarbamoyl, N,N-Diniederalkylcarbamoyl, N-Arylcarbamoyl, Cyano, Mercapto, Niederalkylthio, Niederalkylsulfonyl, Sulfamoyl, N-Niederalkylsulfamoyl und N,N-Diniederalkylsulfamoyl bedeuten, wobei Aryl für Phenyl steht, das unsubstituiert oder durch Niederalkyl, Niederalkoxy, Hydroxy, Halogen oder Trifluormethyl substituiert ist; die Reste $R_3$, $R_4$, $R_6$, $R_8$ und $R_9$ unabhängig voneinander Wasserstoff oder Niederalkyl bedeuten; und $R_5$ und $R_7$

unabhängig voneinander für Wasserstoff, Niederalkyl, Hydroxy, Niederalkoxy, Niederalkanoyloxy, Amino, Niederalkylamino, Diniederalkylamino, $C_4$-$C_7$-Alkylenamino oder Oxa-, Thia- oder Aza-$C_4$-$C_7$-alkylenamino stehen; Tautomeren davon, oder Salzen davon; wobei "Nieder" oder "nieder" einen Rest bis und mit maximal 7 Kohlenstoffatomen bezeichnet; dadurch gekennzeichnet, dass man entsprechend substituierte Ausgangsmaterialien verwendet.

**3.** Verfahren gemäss Anspruch 1 zur Herstellung von Verbindungen der Formel I, worin A eine direkte Bindung oder $-(CH_2)_n-$, worin n für 1 oder 2 steht, bedeutet; X für einen Rest $-C(=Y)-NR_6R_7$ steht; Y für NH, O oder S steht; Z für NH, O oder S steht; $R_1$ und $R_2$ unabhängig voneinander Wasserstoff oder einen oder zwei Substituenten aus der Gruppe bestehend aus Niederalkyl, Trifluormethyl, Phenylniederalkyl, Hydroxy, Niederalkoxy und Halogen bedeuten; die Reste $R_3$, $R_4$ und $R_6$ Wasserstoff bedeuten; und $R_5$ und $R_7$ unabhängig voneinander für Wasserstoff, Niederalkyl, Hydroxy oder Amino stehen; Tautomeren davon, oder Salzen davon; wobei "Nieder" oder "nieder" einen Rest bis und mit maximal 7 Kohlenstoffatomen bezeichnet; dadurch gekennzeichnet, dass man entsprechend substituierte Ausgangsmaterialien verwendet.

**4.** Verfahren gemäss Anspruch 1 zur Herstellung einer Verbindung der Formel I, worin A eine direkte Bindung oder $-CH_2-$ bedeutet; X für einen Rest $-C(=Y)-NR_6R_7$ steht; Y für NH oder S steht; Z für NH steht; $R_1$ Wasserstoff oder einen oder zwei Substituenten aus der Gruppe bestehend aus Niederalkyl, Hydroxy, Niederalkoxy und Halogen bedeutet; $R_2$ für Wasserstoff oder Niederalkyl steht; die Reste $R_3$, $R_4$ und $R_6$ Wasserstoff bedeuten; und $R_5$ und $R_7$ unabhängig voneinander für Wasserstoff, Niederalkyl oder Hydroxy stehen; Tautomeren davon, oder pharmazeutisch anwendbaren Salzen davon; wobei "Nieder" oder "nieder" einen Rest bis und mit maximal 7 Kohlenstoffatomen bezeichnet; dadurch gekennzeichnet, dass man entsprechend substituierte Ausgangsmaterialien verwendet.

**5.** Verfahren gemäss Anspruch 1 zur Herstellung von Verbindungen der Formel I, worin A eine direkte Bindung bedeutet, X für einen Rest $-C(=NH)-NH_2$ steht; Z für NH steht; $R_1$ Wasserstoff oder einen oder zwei Substituenten aus der Gruppe bestehend aus Niederalkyl, Hydroxy und Niederalkoxy bedeutet; die Reste $R_2$, $R_3$ und $R_4$ Wasserstoff bedeuten; und $R_5$ für Wasserstoff oder Hydroxy steht; Tautomeren davon, oder pharmazeutisch anwendbaren Salzen davon; wobei "Nieder" oder "nieder" einen Rest bis und mit maximal 7 Kohlenstoffatomen bezeichnet; dadurch gekennzeichnet, dass man entsprechend substituierte Ausgangsmaterialien verwendet.

**6.** Verfahren gemäss Anspruch 1 zur Herstellung von 4-Amidino-1-indanon-2'-amidinohydrazon, oder einem pharmazeutisch anwendbaren Salz davon; dadurch gekennzeichnet, dass man entsprechend substituierte Ausgangsmaterialien verwendet.

**7.** Verfahren gemäss Anspruch 1 zur Herstellung von 4-Amidino-1-indanon-2'-(N-hydroxyamidino)-hydrazon, oder einem pharmazeutisch anwendbaren Salz davon; dadurch gekennzeichnet, dass man entsprechend substituierte Ausgangsmaterialien verwendet.

**8.** Verfahren gemäss Anspruch 1 zur Herstellung von 5-Amidino-1-tetralon-2'-amidinohydrazon, oder einem pharmazeutisch anwendbaren Salz davon; dadurch gekennzeichnet, dass man entsprechend substituierte Ausgangsmaterialien verwendet.

**9.** Verwendung einer gemäss einem der Ansprüche 1 bis 8 erhaltenen Verbindungen zur Herstellung pharmazeutischer Präparate.

**10.** Verwendung einer gemäss einem der Ansprüche 1 bis 8 erhaltenen Verbindung zur Herstellung pharmazeutischer Präparate für die Behandlung von Krankheiten, die auf eine Hemmung des Enzyms S-Adenosylmethionindecarboxylase ansprechen.

## Claims

**Claims for the following Contracting States : AT, BE, CH, DE, DK, GB, FR, IT, LI, LU, NL, SE**

1. A compound of formula I

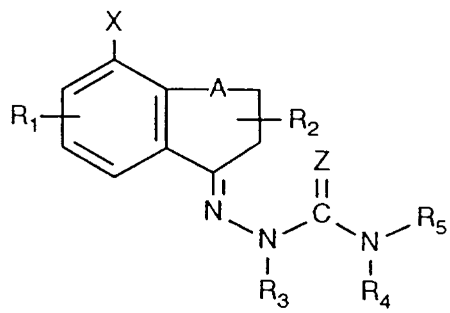

(I)

wherein A is a direct bond or $-(CH_2)_n-$, wherein n is 1, 2 or 3; X is a radical $-C(=Y)-NR_6R_7$; Y is $NR_8$, O or S; Z is $NR_9$, O or S; $R_1$ and $R_2$ are each independently of the other hydrogen or one or more substituents selected from lower alkyl, trifluoromethyl, $C_3$-$C_8$cycloalkyl that is unsubstituted or lower alkyl-substituted, aryl-lower alkyl, hydroxy, lower alkoxy, aryl-lower alkoxy, aryloxy, lower alkanoyloxy, halogen, amino, N-lower alkylamino, N,N-di-lower alkylamino, lower alkanoylamino, nitro, lower alkanoyl, arylcarbonyl, carboxy, lower alkoxycarbonyl, carbamoyl, N-lower alkylcarbamoyl, N,N-di-lower alkylcarbamoyl, N-arylcarbamoyl, cyano, mercapto, lower alkylthio, lower alkylsulfonyl, sulfamoyl, N-lower alkylsulfamoyl and N,N-di-lower alkylsulfamoyl; the radicals $R_3$, $R_4$, $R_6$, $R_8$ and $R_9$ are each independently of the others hydrogen or lower alkyl; and $R_5$ and $R_7$ are each independently of the other hydrogen, lower alkyl, hydroxy, lower alkoxy, lower alkanoyloxy, unsubstituted amino, di-lower alkylamino, $C_4$-$C_7$-alkyleneamino or oxa-, thia- or aza-$C_4$-$C_7$alkyleneamino; a tautomer thereof, or a salt thereof; aryl being phenyl or naphthyl each of which is unsubstituted or substituted by one or more substituents selected from lower alkyl, lower alkoxy, hydroxy, lower alkanoyloxy, nitro, amino, halogen, trifluoromethyl, carboxy, lower alkoxycarbonyl, carbamoyl, N-lower alkylcarbamoyl, N,N-di-lower alkylcarbamoyl, cyano, lower alkanoyl, arylcarbonyl, lower alkylsulfonyl, sulfamoyl, N-lower alkylsulfamoyl and N,N-di-lower alkylsulfamoyl; and the term "lower" or "-lower" indicating a radical having up to and including a maximum of 7 carbon atoms.

2. A compound of formula I according, to claim 1 wherein A is a direct bond or $-(CH_2)_n-$, wherein n is 1 or 2; X is a radical $-C(=Y)-NR_6R_7$; Y is $NR_8$, O or S; Z is $NR_9$, O or S; $R_1$ and $R_2$ are each independently of the other hydrogen or one or two substituents from the group consisting of lower alkyl, trifluoromethyl, cycloalkyl, aryl-lower alkyl, hydroxy, lower alkoxy, aryl-lower alkoxy, aryloxy, lower alkanoyloxy, halogen, amino, N-lower alkylamino, N,N-di-lower alkylamino, lower alkanoylamino, nitro, lower alkanoyl, arylcarbonyl, carboxy, lower alkoxycarbonyl, carbamoyl, N-lower alkylcarbamoyl, N,N-di-lower alkylcarbamoyl, N-arylcarbamoyl, cyano, mercapto, lower alkylthio, lower alkylsulfonyl, sulfamoyl, N-lower alkylsulfamoyl and N,N-di-lower alkylsulfamoyl, aryl being phenyl that is unsubstituted or substituted by lower alkyl, lower alkoxy, hydroxy, halogen or by trifluoromethyl; the radicals $R_3$, $R_4$, $R_6$, $R_8$ and $R_9$ are each independently of the others hydrogen or lower alkyl; and $R_5$ and $R_7$ are each independently of the other hydrogen, lower alkyl, hydroxy, lower alkoxy, lower alkanoyloxy, amino, lower alkylamino, di-lower alkylamino, $C_4$-$C_7$alkyleneamino or oxa-, thia- or aza-$C_4$-$C_7$alkyleneamino; a tautomer thereof, or a salt thereof; the term "lower" or "-lower" indicating a radical having up to and including a maximum of 7 carbon atoms.

3. A compound of formula I according to claim 1 wherein A is a direct bond or $-(CH_2)_n-$, wherein n is 1 or 2; X is a radical $-C(=Y)-NR_6R_7$; Y is NH, O or S; Z is NH, O or S; $R_1$ and $R_2$ are each independently of the other hydrogen or one or two substituents from the group consisting of lower alkyl, trifluoromethyl, phenyl-lower alkyl, hydroxy, lower alkoxy and halogen; the radicals $R_3$, $R_4$ and $R_6$ are hydrogen; and $R_5$ and $R_7$ are each independently of the other hydrogen, lower alkyl, hydroxy or amino; a tautomer thereof, or a salt thereof; the term "lower" or "-lower" indicating a radical having up to and including a maximum of 7 carbon atoms.

**4.** A compound of formula I according to claim 1 wherein A is a direct bond or $-CH_2-$; X is a radical -C(=Y)-$NR_6R_7$; Y is NH or S; Z is NH; $R_1$ is hydrogen or one or two substituents from the group consisting of lower alkyl, hydroxy, lower alkoxy and halogen; $R_2$ is hydrogen or lower alkyl; the radicals $R_3$, $R_4$ and $R_6$ are hydrogen; and $R_5$ and $R_7$ are each independently of the other hydrogen, lower alkyl or hydroxy; a tautomer thereof or a pharmaceutically acceptable salt thereof; the term "lower" or "-lower" indicating a radical having tip to and including a maximum of 7 carbon atoms.

**5.** A compound of formula I according to claim 1 wherein A is a direct bond, X is a radical -C(=NH)-$NH_2$; Z is NH; $R_1$ is hydrogen or one or two substituents from the group consisting of lover alkyl, hydroxy and lover alkoxy; the radicals $R_2$, $R_3$ and $R_4$ are hydrogen; and $R_5$ is hydrogen or hydroxy; a tautomer thereof, or a pharmaceutically acceptable salt thereof; the term "lower" or "-lower" indicating a radical having up to and including a maximum of 7 carbon atoms.

**6.** 4-Amidino-1-indanone-2'-amidinohydrazone according to claim 1 or a pharmaceutically acceptable salt thereof.

**7.** 4-Amidino-1-indanone-2'-(N-hydroxyamidino)-hydrazone according to claim 1 or a pharmaceutically acceptable salt thereof.

**8.** 5-Amidino-1-tetralone-2'-amidinohydrazone according to claim 1 or a pharmaceutically acceptable salt thereof.

**9.** A pharmaceutical composition comprising a compound according to any one of claims 1 to 8 and at least one pharmaceutically acceptable carrier.

**10.** A compound according to any one of claims 1 to 8 for use in a method for the therapeutic treatment of the animal or human body.

**11.** A compound according to any one of claims 1 to 8 for use in the treatment of diseases responsive to inhibition of the enzyme S-adenosylmethionine decarboxylase.

**12.** The use of a compound according to any one of claims 1 to 8 for the manufacture of pharmaceutical compositions.

**13.** The use of a compound according to any one of claims 1 to 8 for the manufacture of pharmaceutical compositions for the treatment of diseases responsive to inhibition of the enzyme S-adenosylmethionine decarboxylase.

**14.** A process for the preparation of a compound of formula I according to claim 1, which process comprises

(a) condensing a compound of formula II

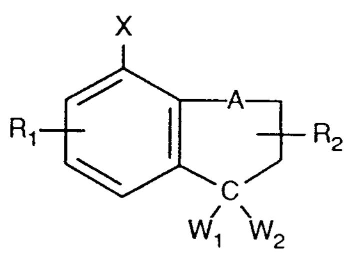

(II)

wherein the group $CW_1W_2$ is carbonyl, functionally modified carbonyl or protected carbonyl and A, X, $R_1$ and $R_2$ are as defined for formula I, with an amine of formula III

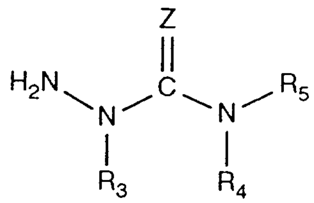

(III)

wherein Z, $R_3$, $R_4$ and $R_5$ are as defined for formula I, or
(b) in a compound of formula IV

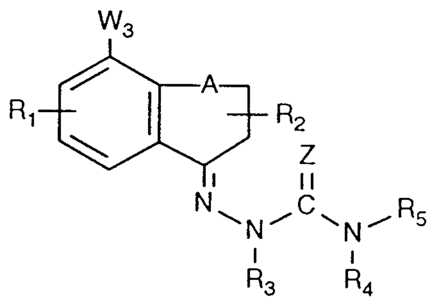

(IV)

wherein $W_3$ is a radical that can be converted into a group X in formula I and A, Z, $R_1$, $R_2$, $R_3$, $R_4$ and $R_5$ are as defined for formula I, converting the radical $W_3$ into the group X; and, if desired, converting a resulting compound of formula I into a different compound of formula I and/or, if desired, converting a resulting salt into the free compound or into a different salt and/or, if desired, converting a resulting free compound of formula I having salt-forming properties into a salt.

**Claims for the following Contracting States : ES, GR**

**1.** A process for the preparation of a compound of formula I

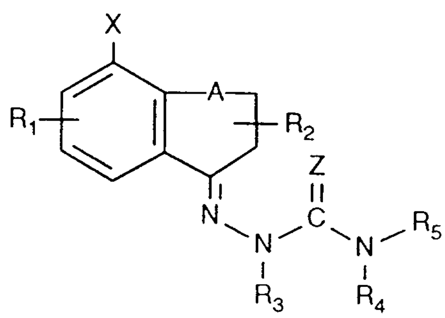

(I)

wherein A is a direct bond or $-(CH_2)_n-$, wherein n is 1, 2 or 3; X is a radical $-C(=Y)-NR_6R_7$; Y is $NR_8$, O or S; Z is $NR_9$, O or S; $R_1$ and $R_2$ are each independently of the other hydrogen or one or more substituents selected from lower alkyl, trifluoromethyl, $C_3$-$C_8$cycloalkyl that is unsubstituted or lower alkyl-substituted, aryl-lower alkyl, hydroxy, lower alkoxy, aryl-lower alkoxy, aryloxy, lower alkanoyloxy, halogen, amino, N-lower alkylamino, N,N-di-lower alkylamino, lower alkanoylamino, nitro, lower alkanoyl, arylcarbonyl, carboxy, lower alkoxycarbonyl, carbamoyl, N-lower alkylcarbamoyl, N,N-di-lower alkylcarbamoyl, N-arylcarbamoyl, cyano, mercapto, lower alkylthio, lower alkylsulfonyl, sulfamoyl, N-lower alkylsulfamoyl and N,N-di-lower alkylsulfamoyl; the radicals $R_3$, $R_4$, $R_6$, $R_8$ and $R_9$ are each independently of the others hydrogen or lower alkyl; and $R_5$ and $R_7$ are each independently of the other hydrogen, lower alkyl, hydroxy, lower alkoxy, lower alkanoyloxy, unsubstituted amino, di-lower alkylamino, $C_4$-$C_7$-alkyleneamino or oxa-, thia- or aza-$C_4$-$C_7$alkyleneamino; a tautomer thereof, or a salt thereof; aryl being phenyl or naphthyl each of which is unsubstituted or substituted by one or more

substituents selected from lower alkyl, lower alkoxy, hydroxy, lower alkanoyloxy, nitro, amino, halogen, trifluoromethyl, carboxy, lower alkoxycarbonyl, carbamoyl, N-lower alkylcarbamoyl, N,N-di-lower alkylcarbamoyl, cyano, lower alkanoyl, arylcarbonyl, lower alkylsulfonyl, sulfamoyl, N-lower alkylsulfamoyl and N,N-di-lower alkylsulfamoyl; and the term "lower" or "-lower" indicating a radical having up to and including a maximum of 7 carbon atoms; which process comprises

(a) condensing a compound of formula II

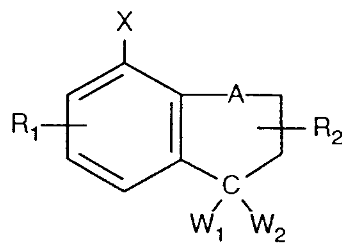

(II)

wherein the group $CW_1W_2$ is carbonyl, functionally modified carbonyl or protected carbonyl and A, X, $R_1$ and $R_2$ are as defined for formula I, with an amine of formula III

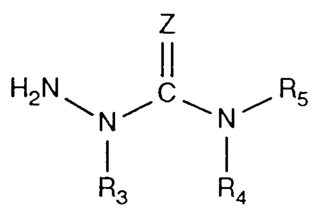

(III)

wherein Z, $R_3$, $R_4$ and $R_5$ are as defined for formula I, or

(b) in a compound of formula IV

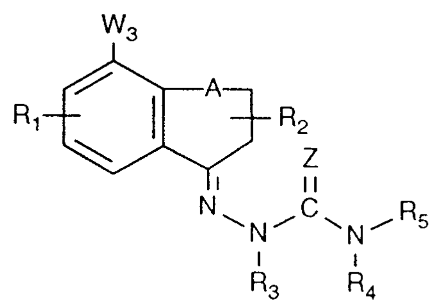

(IV)

wherein $W_3$ is a radical that can be converted into a group X in formula I and A, Z, $R_1$, $R_2$, $R_3$, $R_4$ and $R_5$ are as defined for formula I, converting the radical $W_3$ into the group X; and, if desired, converting a resulting compound of formula I into a different compound of formula I and/or, if desired, converting a resulting salt into the free compound or into a different salt and/or, if desired, converting a resulting free compound of formula I having salt-forming properties into a salt.

**2.** A process according to claim 1 for the preparation of a compound of formula I wherein A is a direct bond or $-(CH_2)_n-$, wherein n is 1 or 2; X is a radical $-C(=Y)-NR_6R_7$; Y is $NR_8$, O or S; Z is $NR_9$, O or S; $R_1$ and $R_2$ are each independently of the other hydrogen or one or two substituents from the group consisting of lower alkyl, trifluoromethyl, cycloalkyl, aryl-lower alkyl, hydroxy, lower alkoxy, aryl-lower alkoxy, aryloxy, lower alkanoyloxy, halogen, amino, N-lower alkylamino, N,N-di-lower alkylamino, lower alkanoylamino, nitro, lower alkanoyl, arylcarbonyl, carboxy, lower alkoxycarbonyl, carbamoyl, N-lower alkylcarbamoyl, N,N-di-lower alkylcarbamoyl, N-arylcarbamoyl, cyano, mercapto, lower alkylthio, lower alkylsulfonyl, sulfamoyl, N-lower alkylsulfamoyl and N,N-di-lower alkylsulfamoyl, aryl being phenyl that

is unsubstituted or substituted by lower alkyl, lower alkoxy, hydroxy, halogen or by trifluoromethyl; the radicals $R_3$, $R_4$, $R_6$, $R_8$ and $R_9$ are each independently of the others hydrogen or lower alkyl; and $R_5$ and $R_7$ are each independently of the other hydrogen, lower alkyl, hydroxy, lower alkoxy, lower alkanoyloxy, amino, lower alkylamino, di-lower alkylamino, $C_4$-$C_7$alkyleneamino or oxa-, thia- or aza-$C_4$-$C_7$alkyleneamino; a tautomer thereof, or a salt thereof; the term "lower" or "-lower" indicating a radical having up to and including a maximum of 7 carbon atoms, in which process appropriately substituted starting materials are used.

**3.** A process according to claim 1 for the preparation of a compound of formula I wherein A is a direct bond or $-(CH_2)_n-$, wherein n is 1 or 2; X is a radical $-C(=Y)-NR_6R_7$; Y is NH, O or S; Z is NH, O or S; $R_1$ and $R_2$ are each independently of the other hydrogen or one or two substituents from the group consisting of lower alkyl, trifluoromethyl, phenyl-lower alkyl, hydroxy, lower alkoxy and halogen; the radicals $R_3$, $R_4$ and $R_6$ are hydrogen; and $R_5$ and $R_7$ are each independently of the other hydrogen, lower alkyl, hydroxy or amino; a tautomer thereof, or a salt thereof; the term "lower" or "-lower" indicating a radical having up to and including a maximum of 7 carbon atoms, in which process appropriately substituted starting materials are used.

**4.** A process according to claim 1 for the preparation of a compound of formula I wherein A is a direct bond or $-CH_2-$; X is a radical $-C(=Y)-NR_6R_7$; Y is NH or S; Z is NH; $R_1$ is hydrogen or one or two substituents front the group consisting of lower alkyl, hydroxy, lower alkoxy and halogen; $R_2$ is hydrogen or lower alkyl; the radicals $R_3$, $R_4$ and $R_6$ are hydrogen; and $R_5$ and $R_7$ are each independently of the other hydrogen, lower alkyl or hydroxy; a tautomer thereof, or a pharmaceutically acceptable salt thereof; the term "lower" or "-lower" indicating a radical having up to and including a maximum of 7 carbon atoms, in which process appropriately substituted starting materials are used.

**5.** A process according to claim 1 for the preparation of a compound of formula I wherein A is a direct bond, X is a radical $-C(=NH)-NH_2$; Z is NH; $R_1$ is hydrogen or one or two substituents from the group consisting of lower alkyl, hydroxy and lower alkoxy; the radicals $R_2$, $R_3$ and $R_4$ are hydrogen; and $R_5$ is hydrogen or hydroxy; a tautomer thereof, or a pharmaceutically acceptable salt thereof; the term "lower" or "-lower" indicating a radical having up to and including a maximum of 7 carbon atoms, in which process appropriately substituted starting materials are used.

**6.** A process according to claim 1 for the preparation of 4-amidino-1-indanone-2'-amidinohydrazone or a pharmaceutically acceptable salt thereof, in which process appropriately substituted starting materials are used.

**7.** A process according to claim 1 for the preparation of 4-amidino-1-indanone-2'-(N-hydroxyamidino)-hydrazone or a pharmaceutically acceptable salt thereof, wherein correspondingly substituted starting materials are used.

**8.** A process according to claim 1 for the preparation of 5-amidino-1-tetralone-2'-amidinohydrazone or a pharmaceutically acceptable salt thereof, wherein correspondingly substituted starting materials are used.

**9.** The use of a compound obtained in accordance with any one of claims 1 to 8 for the manufacture of pharmaceutical compositions.

**10.** The use of a compound obtained in accordance with any one of claims 1 to 8 for the manufacture of pharmaceutical compositions for the treatment of diseases responsive to inhibition of the enzyme S-adenosylmethionine decarboxylase.

## Revendications

**Revendications pour les Etats contractants suivants : AT, BE, CH, DE, DK, FR, GB, IT, LI, LU, NL, SE**

1. Composés de formule I

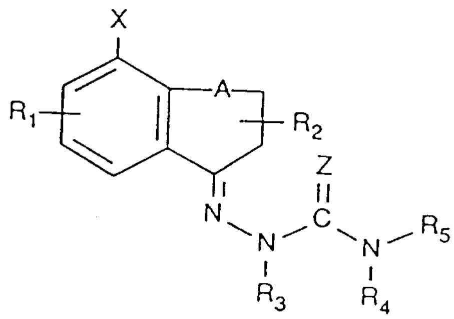

(I)

dans laquelle A représente une liaison directe ou $-(CH_2)_n-$ dans lequel n est égal à 1, 2 ou 3 ; X représente un groupe $-C(=Y)-NR_6R_7$ ; Y représente $NR_8$, 0 ou S ; Z représente NRg, O ou S ; $R_1$ et $R_2$ représentent chacun, indépendamment l'un de l'autre, l'hydrogène ou un ou plusieurs substituants choisis parmi les groupes alkyle inférieur, trifluorométhyle, cycloalkyle en $C_3$-$C_8$ non substitué ou substitué par un groupe alkyle inférieur, aryl-alkyle inférieur, hydroxy, alcoxy inférieur, aryl-alcoxy inférieur, aryloxy, alcanoyloxy inférieur, les halogènes, les groupes amino, N-(alkyle inférieur)-amino, N,N-di-(alkyle inférieur)-amino, alcanoylamino inférieur, nitro, alcanoyle inférieur, arylcarbonyle, carboxy, (alcoxy inférieur)-carbonyle, carbamoyle, N-(alkyle inférieur)-carbamoyle, N,N-di-(alkyle inférieur)-carbamoyle, N-arylcarbamoyle, cyano, mercapto, alkylthio inférieur, alkylsulfonyÉ inférieur, sulfamoyle, N-(alkyle inférieur)-sulfamoyle, et N,N-di-(alkyle inférieur)-sulfamoyle ; $R_3$, $R_4$, $R_6$, $R_8$ et $R_9$ représentent chacun, indépendamment les uns des autres, l'hydrogène ou un groupe alkyle inférieur ; et $R_5$ et $R_7$ représentent chacun, indépendamment l'un de l'autre, l'hydrogène, un groupe alkyle inférieur, hydroxy, alcoxy inférieur, alcanoyloxy inférieur, amino non substitué, di(alkyle inféneur)-amino, (alkylène en $C_4$-$C_7$)-amino ou oxa-, thia- ou aza-(alkylène en $C_4$-$C_7$)-amino ; leurs tautomères ou leurs sels ; un groupe aryle étant un groupe phényle ou naphtyle non substitué ou portant un ou plusieurs substituants choisis parmi les groupes alkyle inférieur, alcoxy inférieur, hydroxy, alcanoyloxy inférieur, nitro, amino, les halogènes, les groupes trifluorométhyle, carboxy, (alcoxy inférieur)-carbonyle, carbamoyle, N-(alkyle inférieur)-carbamoyle, N,N-di-(alkyle inférieur)-carbamoyle, cyano, alcanoyle inférieur, arylcarbonyle, alkylsulfonyle inférieur, sulfamoyle, N-(alkyle inférieur)-sulfamoyle et N,N-di-(alkyle inférieur)-sulfamoyle ; et l'expression "inférieur" s'appliquant à des groupes qui contiennent au maximum jusqu'à 7 atomes de carbone.

2. Composés de formule I selon revendication 1, dans laquelle A représente une liaison directe ou $-(CH_2)_n$ dans lequel n est égal à 1 ou 2 ; X représente un groupe $-C(=Y)NR_6R_7$ ; Y représente $NR_8$, 0 ou S ; Z représente $NR_9$ , O ou S ; $R_1$ et $R_2$ représentent chacun, inpendamment l'un de l'autre, l'hydrogène ou un ou deux substituants choisis parmi les groupes alkyle inférieur, trifluorométhyle, cycloalkyle, aryl-alkyle inférieur, hydroxy, alcoxy inférieur, aryl-alcoxy inférieur, aryloxy, alcanoyloxy inférieur, les halogènes, les groupes amino, N-(alkyle inférieur)-amino, N,N-di-(alkyle inférieur)-amino, alcanoylamino inférieur, nitro, alcanoyle inférieur, arylcarbonyle, carboxy, (alcoxy inférieur)-carbonyle, carbamoyle, N-(alkyle inférieur)-carbamoyle, N,N-di-(alkyle inférieur)-carbamoyle, N-arylcarbamoyle, cyano, mercapto, alkylthio inférieur, alkylsulfonyle inférieur, sulfamoyle, N-(alkyle inférieur)-sulfamoyle et N,N-di-(alkyle inférieur)-sulfamoyle, un groupe aryle étantun groupe phényle non substitué ou substitué par des groupes alkyle inférieur, alcoxy inférieurf, hydroxy, des halogènes ou des groupes trifluorométhyle ; $R_3$, $R_4$, $R_6$, $R_8$ et $R_9$ représentent chacun, indépendamment les uns des autres, l'hydrogène ou un groupe alkyle ifnérieur ; et $R_5$ et $R_7$ représentent chacun, indépendamment l'un de l'autre, l'hydrogène, un groupe alkyle inférieur, hydroxy, alcoxy inférieur, alcanoyloxy inférieur, amino, alkylamino inférieur, di-(alkyle inférieur)-amino, (alkylène en $C_4$-$C_7$)-amino ou oxa-, thia- ou aza-(alkylène en $C_4$-$C_7$)-amino ; leurs tautomères ou leurs sels ; l'apression "inférieur" s appliquant à des groupes qui contiennent jusqu'à 7 atomes de carbone au maximum.

3. Composés de formule I selon revendication 1, dans laquelle A représente une liaison directe ou $-(CH_2)$-dans lequel n est égal à 1 ou 2 ; X représente un groupe $-C(=Y)-NR_6R_7$ ; Y représente NH, O ou S ; Z représente NH, O ou S ; $R_1$ et $R_2$ représentent chacun, indépendamment l'un de l'autre, l'hydrogène ou un ou deux substituants choisis parmi les groupes alkyle inférieur, trifluorométhyle, phényl-alkyle inférieur, hydroxy, alcoxy inférieur et les halogènes ; $R_3$, $R_4$ et $R_6$ représentent l'hydrogène ; et $R_5$ et $R_7$ représentent chacun, indépendamment l'un de l'autre, l'hydrogène, un groupe alkyle inférieur, hydroxy ou amino ; leurs tautomères ou leurs sels ; l'expression "inférieur" s'appliquant à des groupes qui contiennent jusqu'à 7 atomes de carbone au maximum.

4. Composé de formule I selon revendication 1, dans laquelle A représente une liaison directe ou $-CH_2-$ ; X représente un groupe $-C(=Y)-NR_6R_7$ ; Y représente NH ou S ; Z représente NH ; $R_1$ représente l'hydrogène ou un ou deux substituants choisis parmi les groupes alkyle inférieur, hydroxy, alcoxy inférieur et les halogènes ; $R_2$ représente l'hydrogène ou un groupe alkyle inférieur ; $R_3$, $R_4$ et $R_6$ représentent l'hydrogène ; et $R_5$ et $R_7$ représentent chacun indépendamment l'un de l'autre, l'hydrogène, un groupe alkyle inférieur ou hydroxy ; leurs tautomères ou leurs sels acceptables pour l'usage pharmaceutique ; l'expression "inférieur" s appliquant à un groupe qui contient au maximum 7 atomes de carbone.

5. Composés de formule I selon revendication 1, dans laquelle A représente une liaison directe, X représente un groupe $-C(=NH)-NH_2$ ; Z représente NH ; $R_1$ représente l'hydrogène ou un ou deux substituants choisis parmi les groupes alkyle inférieur/, hydroxy et alcoxy inférieur ; $R_2$, $R_3$ et $R_4$ représentent l'hydrogène ; et $R_5$ représente l'hydrogène ou un groupe hydroxy ; leurs tautomères ou leurs sels acceptables pour l'usage pharmaceutique ; l'expression "inférieur" s'appliquant à un groupe qui contient au maximum 7 atomes de carbone.

6. La 4-amidino-1-indanone-2'-amidinohydrazone selon la revendication 1, ou l'un de ses sels acceptables pour l'usage pharmaceutique.

7. La 4-amidino-1-indanone-2'-(N-hydroxyamidino)-hydrazone selon la revendication 1 ou l'un de ses sels acceptables pour l'usage pharamceutique.

8. La 5-amidino-1-tétralone-2'-amidinohydrazone selon la revendication 1, ou l'un de ses sels acceptables pour l'usage pharmaceutique.

9. Compositions pharmaceutiques contenant un composé selon l'une des revendications 1 à 8 et au moins un véhicule acceptable pour l'usage pharmaceutique.

10. Un composé selon l'une des revendications 1 à 8 pour l'utilisation dans un procédé pour le traitement thérapeutique de l'organisme animal ou humain.

11. Un composé selon l'une des revendications 1 à 8, pour l'utilisation dans le traitement de maladies demandant une inhibition de l'enzyme S-adénosylméthioninedécarboxylase.

12. Utilisation d'un composé selon l'une des revendications 1 à 8 pour la préparation de compositions pharmaceutiques.

13. Utilisation d'un composé selon l'une des revendications 1 à 8 pour la préparation de compositions pharmaceutiques prévues pour le traitement de maladies demandant une inhibition de l'enzyme S-adénosylméthioninedécarboxylase.

14. Procédé de préparation d'un composé de formule I selon la revendication 1, caractérisé en ce que

(a) on condense un composé de formule II

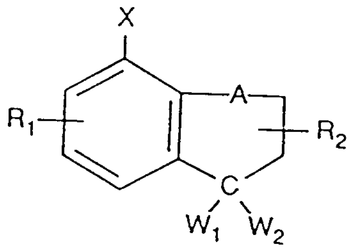

(II)

dans laquelle le groupe $CW_1W_2$ est un groupe carbonyle, un groupe carbonyle qui a subi une modification fonctionnelle ou un groupe carbonyle protégé et A, X $R_1$ et $R_2$ ont les significations indiquées en référence à la formule I, avec une amine de formule III

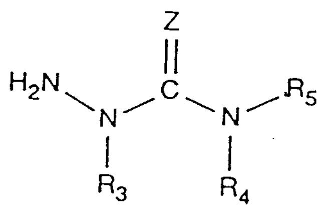

(III)

dans laquelle Z, $R_3$, $R_4$ et $R_5$ ont les significations indiquées en référence à la formule I, ou bien
(b) dans un composé de formule IV

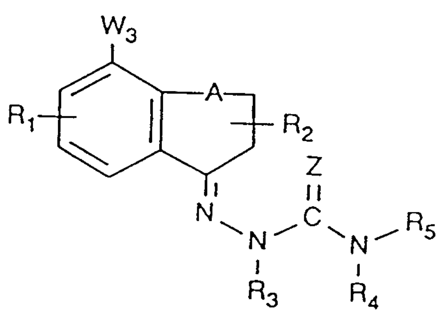

(IV)

dans laquelle $W_3$ représente un substituant qui peut être converti en un groupe X de la formule I, et A, Z, $R_1$, R2, $R_3$, R4 et $R_5$ ont les significations indiquées en référence à la formule I, on convertit le substituant $W_3$ en le groupe X ; et si on le désire, on convertit un composé ainsi obtenu, répondant à la formule I, en un autre composé de formule I et/ou, si on le désire, on convertit un sel ainsi obtenu en le composé libre ou en un autre sel, et/ou si on le désire, on convertit un composé de formule I obtenu à l'état libre et salifiable en un sel.

**Revendications pour les Etats contractants suivants : ES, GR**

**1.** Procédé de préparation des composés de formule I

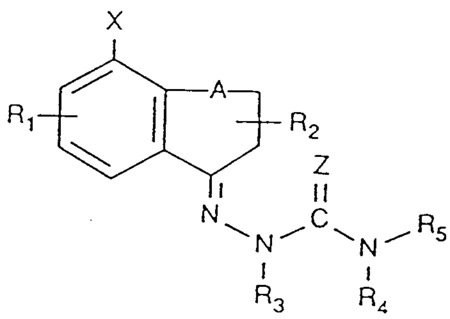

(I)

dans laquelle A représente une liaison directe ou $-(CH_2)_n-$ dans lequel n est égal à 1, 2 ou 3 ; X représente un groupe $-C(=Y)-NR_6R_7$ ; Y représente $NR_8$, 0 ou S ; Z représente NRg, O ou S ; $R_1$ et $R_2$ représentent chacun, indépendamment Itun de Itautre, Ithydrogène ou un ou plusieurs substituants choisis parmi les groupes alkyle inférieur/, trifluorométhyle, cycloalkyle en $C_3$-$C_8$ non substitué ou substitué par un groupe alkyle inférieur, aryl-alkyle inférieur, hydroxy, alcoxy inférieur, aryl-alcoxy inférieur, aryloxy, alcanoyloxy inférieur, les halogènes, les groupes amino, N-(alkyle inférieur)-amino, N,N-di-(alkyle inférieur)-amino, alcanoylamino inférieur, nitro, alcanoyle inférieur, arylcarbonyle, carboxy, (alcoxy inférieur)-carbonyle, carbamoyle, N-(alkyle inférieur)-carbamoyle, N,N-di-(alkyle inférieur)-carbamoyle, N-arylcarbamoyle, cyano, mercapto, alkylthio inférieur, alkylsulfonyÉ inférieur, sulfamoyle, N-(alkyle inférieur)-sulfamoyle, et N,N-di-(alkyle inférieur)-sulfamoyle ; $R_3$, $R_4$, $R_6$, $R_8$ et $R_9$ représentent chacun, indépendamment les uns des autres, l'hydrogène ou un groupe alkyle inférieur ; et $R_5$ et $R_7$ représentent chacun, indépendamment l'un de l'autre, l'hydrogène, un groupe alkyle inférieur, hydroxy, alcoxy inférieur, alcanoyloxy inférieur, amino non substitué, di(alkyle inféneur)-amino, (alkylène en $C_4$-$C_7$)-amino ou oxa-, thia- ou aza-(alkylène en $C_4$-$C_7$)-amino ; leurs tautomères ou leurs sels ; un groupe aryle étant un groupe phényle ou naphtyle non substitué ou portant un ou plusieurs substituants choisis parmi les groupes alkyle inférieur, alcoxy inférieur, hydroxy, alcanoyloxy inférieur, nitro, amino, les halogènes, les groupes trifluorométhyle, carboxy, (alcoxy inférieur)-carbonyle, carbamoyle, N-(alkyle inférieur)-carbamoyle, N,N-di-(alkyle inférieur)-carbamoyle, cyano, alcanoyle inférieur, arylcarbonyle, alkylsulfonyle inférieur, sulfamoyle, N-(alkyle inférieur)-sulfamoyle et N,N-di-(alkyle inférieur)-sulfamoyle ; et l'expression "inférieur" s'appliquant à des groupes qui contiennent au maximum jusqu'à 7 atomes de carbone caractérisé en ce que

(a) on condense un composé de formule II

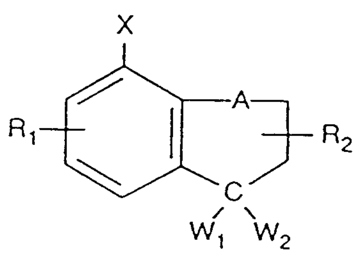

(II)

dans laquelle le groupe $CW_1W_2$ est un groupe carbonyle, un groupe carbonyle qui a subi une modification fonctionnelle ou un groupe carbonyle protégé et A, X, $R_1$ et $R_2$ ont les significations indiquées en référence à la formule I, avec une amine de formule III

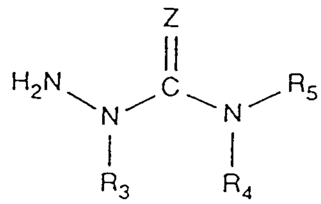

(III)

dans laquelle Z, $R_3$, R4 et $R_5$ ont les significations indiquées en référence à la formule I, ou bien (b) dans un composé de formule IV

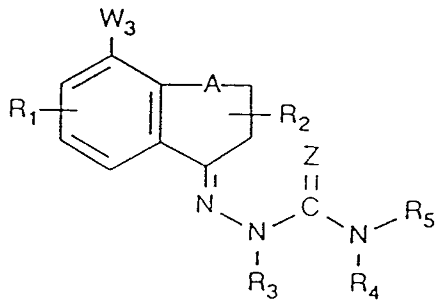

(IV)

dans laquelle $W_3$ représente un substituant qui peut être converti en un groupe X de la formule I, et A, Z, $R_1$, R2, $R_3$, R4 et $R_5$ ont les significations indiquées en référence à la formule I, on convertit le substituant $W_3$ en le groupe X ; et si on le désire, on convertit un composé ainsi obtenu, répondant à la formule I, en un autre composé de formule I et/ou, si on le désire, on convertit un sel ainsi obtenu en le composé libre ou en un autre sel, et/ou si on le désire, on convertit un composé de formule I obtenu à l'état libre et salifiable en un sel.

**2.** Procédé selon revendication 1, pour la préparation des composés de formule I dans laquelle A représente une liaison directe ou $-(CH_2)-_n$ dans lequel n est égal à 1 ou 2 ; X représente un groupe -C-(=Y)$NR_6R_7$ ; Y représente $NR_8$, 0 ou S ; Z représente $NR_9$, O ou S ; $R_1$ et $R_2$ représentent chacun, inpendamment l'un de l'autre, l'hydrogène ou un ou deux substituants choisis parmi les groupes alkyle inférieurf, trifluorométhyle, cycloalkyle, aryl-alkyle inférieur, hydroxy, alcoxy inférieur, aryl-alcoxy inférieur, aryloxy, alcanoyloxy inférieur, les halogènes, les groupes amino, N-(alkyle inférieur)-amino, N,N-di-(alkyle inférieur)-amino, alcanoylamino inférieur, nitro, alcanoyle inférieur, arylcarbonyle, carboxy, (alcoxy inférieur)-carbonyle, carbamoyle, N-(alkyle inférieur)-carbamoyle, N,N-di-(alkyle inférieur)-carbamoyle, N-arylcarbamoyle, cyano, mercapto, alkylthio inférieur, alkylsulfonyle inférieur, sulfamoyle, N-(alkyle inférieur)-sulfamoyle et N,N-di-(alkyle inférieur)-sulfamoyle, un groupe aryle étantun groupe phényle non substitué ou substitué par des groupes alkyle inférieur, alcoxy inférieurf, hydroxy, des halogènes ou des groupes trifluorométhyle ; $R_3$, $R_4$, $R_6$, $R_8$ et $R_9$ représentent chacun, indépendamment les uns des autres, l'hydrogène ou un groupe alkyle ifnérieur ; et $R_5$ et $R_7$ représentent chacun, indépendamment l'un de l'autre, l'hydrogène, un groupe alkyle inférieur, hydroxy, alcoxy inférieur, alcanoyloxy inférieur, amino, alkylamino inférieur, di-(alkyle inférieur)-amino, (alkylène en $C_4$-$C_7$)-amino ou oxa-, thia- ou aza-(alkylène en $C_4$-$C_7$)-amino ; leurs tautomères ou leurs sels ; l'apression "inférieur" s'appliquant à des groupes qui contiennent jusqu'à 7 atomes de carbone au maximum ; caractérisé en ce que l'on utilise les produits de départ portant les substituants correspondants.

**3.** Procédé selon revendication 1 pour la préparation des composés de formule I dans laquelle A représente une liaison directe ou $-(CH_2)-$ dans lequel n est égal à 1 ou 2 ; X représente un groupe -C-(=Y)-$NR_6R_7$ ; Y représente NH, O ou S ; Z représente NH, O ou S ; $R_1$ et $R_2$ représentent chacun, indépendamment l'un de l'autre, l'hydrogène ou un ou deux substituants choisis parmi les groupes alkyle inférieur, trifluorométhyle, phényl-alkyle inférieur, hydroxy, alcoxy inférieur et les halogènes ; $R_3$, $R_4$ et $R_6$ représentent l'hydrogène ; et $R_5$ et $R_7$ représentent chacun, indépendamment l'un de l'autre, l'hydrogène, un groupe alkyle inférieur, hydroxy ou amino ; leurs tautomères ou leurs sels ; l'expression "inférieur" s appliquant à des groupes qui contiennent jusqu'à 7 atomes de carbone au maximum ; caractérisé en ce que l'on utilise les produits de départ portant les substituants correspondants.

**4.** Procédé selon revendication 1 pour la préparation d'un composé de formule I dans laquelle A représente une liaison directe ou $-CH_2-$ ; X représente un groupe $-C(=Y)-NR_6R_7$ ; Y représente NH ou S ; Z représente NH ; $R_1$ représente l'hydrogène ou un ou deux substituants choisis parmi les groupes alkyle inférieur, hydroxy, alcoxy inférieur et les halogènes ; $R_2$ représente l'hydrogène ou un groupe alkyle inférieur ; $R_3$, $R_4$ et $R_6$ représentent l'hydrogène ; et $R_5$ et $R_7$ représentent chacun indépendamment l'un de l'autre, l'hydrogène, un groupe alkyle inférieur ou hydroxy ; leurs tautomères ou leurs sels acceptables pour l'usage pharmaceutique ; l'expression "inférieur" s'appliquant à un groupe qui contient au maximum 7 atomes de carbone ; caractérisé en ce que l'on utilise des produits de départ portant les substituants correspondants.

**5.** Procédé selon revendication 1 pour la préparation des composés de formule I dans laquelle A représente une liaison directe, X représente un groupe $-C(=NH)-NH_2$ ; Z représente NH ; $R_1$ représente l'hydrogène ou un ou deux substituants choisis parmi les groupes alkyle inférieur/, hydroxy et alcoxy inférieur ; $R_2$, $R_3$ et $R_4$ représentent l'hydrogène ; et $R_5$ représente l'hydrogène ou un groupe hydroxy ; leurs tautomères ou leurs sels acceptables pour l'usage pharmaceutique ; l'expression "inférieur" s'appliquant à un groupe qui contient au maximum 7 atomes de carbone ; caractérisé en ce que l'on utilise Aes produits de départ portant les substituants correspondants.

**6.** Procédé selon revendication 1 pour la préparation de la 4-amidino-1-indanone-2'-amidinohydrazone ou de l'un de ses sels acceptables pour l'usage pharmaceutique, caractérisé en ce que l'on utilise des produits de départ portant les substituants correspondants.

**7.** Procédé selon revendication 1 pour la préparation de la 4-amidino-1-indanone-2'-(N-hydroxyamidino)-hydrazone ou de l'un de ses sels acceptables pour l'usage pharmaceutique, caractérisé en ce que l'on utilise des produits de départ portant les substituants correspondants.

**8.** Procédé selon revendication 1, pour la préparation de la 5-amidino-1-tétralone-2'-amidinohydrazone ou de l'un de ses sels acceptables pour l'usage pharmaceutique, caractérisé en ce que l'on utilise des produits de départ portant les substituants correspondants.

**9.** Utilisation d'un composé obtenu selon l'une des revendications 1 à 8 pour la préparation de compositions pharanceutiques.

**10.** Utilisation d'un composé obtenu selon l'une des revendications 1 à 8 pour la préparation de compositions pharmaceutiques prévues pour le traitement de maladies demandant une inhibition de l'enzyme S-adénosvlméthioninedécarboxylase.